(19) Europäisches Patentamt European Patent Office Office européen des brevets

(11) **EP 1 872 707 A1**

(12) **EUROPEAN PATENT APPLICATION**

(43) Date of publication:
**02.01.2008 Bulletin 2008/01**

(21) Application number: **07011871.6**

(22) Date of filing: **18.06.2007**

(51) Int Cl.:
*A61B 1/005* (2006.01)  *A61B 5/06* (2006.01)
*A61B 8/12* (2006.01)  *A61B 5/055* (2006.01)
*A61B 6/03* (2006.01)

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **29.06.2006 JP 2006180435**

(71) Applicant: **OLYMPUS MEDICAL SYSTEMS CORP. Tokyo (JP)**

(72) Inventors:
• **Kawashima, Tomonao**
**Tokyo 151-0072 (JP)**
• **Ikuma, Soichi**
**Tokyo 151-0072 (JP)**
• **Obata, Saori**
**Tokyo 151-0072 (JP)**
• **Komuro, Masahiko**
**Tokyo 151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

(54) **Body cavity probe apparatus**

(57) A body cavity probe apparatus can minimally invasively detect the insertion shape of a body cavity probe and the direction of real-time images to create guide images each containing both the insertion shape of the body cavity probe and the direction of the real-time image. An ultrasonic endoscope 2 as a body cavity probe inserted into the body cavity has an ultrasonic transducer array 29 in a rigid portion 21 located at a distal end thereof, to acquire an ultrasonic echo signal, an image position and orientation detecting coil 31 provided in the vicinity of the ultrasonic transducer array 29, and an insertion shape detecting coil 32 provided in a longitudinal direction of the flexible portion 22, thus generating guide images each containing the shape of the flexible portion 22 and the direction of an ultrasonic tomogram generated from the echo signal as a real-time image.

FIG.1

EP 1 872 707 A1

**Description**

BACKGROUND OF THE INVENTION

1. Field of the Invention

The present invention relates to a body cavity probe apparatus to perform diagnosis of the body cavity or the like using a body cavity probe inserted into the body cavity.

2. Description of the Related Art

Conventionally, body cavity probes such as an endoscope, an ultrasonic endoscope, and a small-diameter ultrasonic probe are well-known which are inserted into the body cavity such as the digestive tract, bile duct, pancreatic duct, or vessel and used for diagnosis or treatment. These body cavity probes normally have an image pickup device such as a CCD camera, or an ultrasonic transducer at the distal end.

These body cavity probes are normally used as body cavity probe apparatuses integrated with a processor that creates optical images or ultrasonic tomogram images from signals obtained from the image pickup device or ultrasonic transducer.

Moreover, body cavity probe apparatuses have been known which comprise a navigation function for assisting the body cavity probe so that the probe can easily reach a target site.

A first conventional example of these body cavity probe apparatuses is an ultrasonic diagnosis apparatus disclosed in Japanese Patent Laid-Open No. 2004-113629 and which generates ultrasonic images from ultrasonic signals obtained by transmitting and receiving ultrasonic waves to and from a subject. The ultrasonic diagnosis apparatus comprises ultrasonic scan position detecting means for detecting the position of a site to and from which ultrasonic waves are transmitted and received, ultrasonic image generating means for generating ultrasonic images on the basis of ultrasonic signals, and control means for obtaining anatomical image information on the subject's site corresponding to positional information obtained by the ultrasonic scan position detecting means, from image information holding means having schematic diagram data on the human body as guide images to display the information on the same screen as the ultrasonic image.

The body cavity probe apparatus displays ultrasonic images as real-time images. The body cavity probe apparatus uses a transmission coil that generates magnetic fields and a reception coil that receives magnetic fields to actually detect positional information. One of the coils is provided at an insertion end of the ultrasonic endoscope serving as a body cavity probe, whereas the other coil is installed in the subject. Thus, the body cavity probe apparatus can detect the posture of the subject and thus the position of the subject's site to and from which ultrasonic waves are transmitted and received.

Meanwhile, a second conventional example of the body cavity probe apparatus is an endoscope apparatus disclosed in Japanese Patent Laid-Open No. 2002-306403 and which detects the insertion shape of the endoscope to obtain a video signal from which the insertion shape is extracted. The endoscope apparatus has image generating means for generating a 3-dimensional image of the subject from consecutive slice tomogram images of 3-dimensional regions obtained by CT scanning in advance of the subject and display means for synthesizing the insertion shape with the 3-dimensional image of the subject around the insertion shape to display the result.

The body cavity probe apparatus displays an endoscope image as a real-time image. To actually detect the insertion shape, the body cavity probe apparatus further uses a radioactive substance filled in a flexible tube in the endoscope to radiate γ rays, and a bottom detecting portion and a vertical detecting portion each having a combination of a scintillator that absorbs γ rays to emit light and a light receiving device.

The above-mentioned Japanese Patent Laid-Open No. 2004-113629 describes the capability of creating guide images containing the insertion shape of the body cavity probe and the direction of real-time images (in Japanese Patent Laid-Open No. 2004-113629, ultrasonic images). However, Japanese Patent Laid-Open No. 2004-113629 does not include a sufficient configuration for achieving the capability as described below.

First, Japanese Patent Laid-Open No. 2004-113629 does not describe any configuration for detecting the insertion shape. Thus, disadvantageously, no guide image containing the insertion shape can be created.

Second, Japanese Patent Laid-Open No. 2004-113629 does not describe any configuration for detecting the direction of real-time images. In particular, Japanese Patent Laid-Open No. 2004-113629 does not describe any method for mounting a coil provided at the insertion end of the body cavity probe located at the tip of the apparatus, any output contents, or any degree of freedom of detection. This makes it impossible to detect in which direction a scan surface faces with respect to the body cavity probe.

Thus, a problem with Japanese Patent Laid-Open No. 2004-113629 is that no guide image containing the direction of real-time images can be created. When the electronic convex scanning ultrasonic endoscope is adopted as a body cavity probe described in Japanese Patent Laid-Open No. 2004-113629, the above problem is significant particularly because the angle of torsion around an insertion shaft cannot be detected.

Thus, when an assumption is made that a body cavity probe apparatus is provided by combining Japanese Patent Laid-Open No. 2004-113629 with Japanese Patent Laid-Open No. 2002-306403, the above problems can be described as follows.

For the first problem that no guide image containing the insertion shape can be created, the combination with the body cavity probe apparatus disclosed in Japanese Patent Laid-Open No. 2002-306403 provides a configuration for detecting the insertion shape. This enables the creation of guide images containing the insertion shape.

However, since the body cavity probe apparatus disclosed in Japanese Patent Laid-Open No. 2002-306403 uses $\gamma$ rays to detect the insertion shape, a new problem arises; both operator and subject are likely to be invasively exposed to radiations.

For the second problem that no guide image containing the direction of real-time images can be created, Japanese Patent Laid-Open No. 2002-306403 neither describes any configuration for detecting the direction of real-time images. Thus, Japanese Patent Laid-Open No. 2002-306403 neither solves this problem.

SUMMARY OF THE INVENTION

A first object of the present invention is to provide a body cavity probe apparatus that can minimally invasively detect the insertion shape of a body cavity probe and the direction of real-time images to create guide images containing both of the shape and the direction. A second object of the present invention is to provide a body cavity probe apparatus that can create guide images containing the insertion shape of a body cavity probe and the direction of real-time images.

A body cavity probe apparatus in accordance with the present invention comprises a body cavity probe that is inserted into a body cavity in a subject, insertion shape creation means for creating the insertion shape of the body cavity probe, 3-dimensional image creation means for creating a 3-dimensional image of a human body from 3-dimensional data on the human body, and synthesis means for synthesizing the insertion shape and the 3-dimensional image, the body cavity probe comprising a rigid portion having image signal acquisition means fixed on a side thereof which is inserted into the body cavity, to acquire a signal from which an image of the interior of the subject is created and a flexible portion located closer to a proximal end than the rigid portion, and comprising image creation means for creating a real-time image of the interior of the subject from the signal acquired by the image signal acquiring means, image position and orientation detecting device the position of which is fixed to the rigid portion, a plurality of insertion shape detecting devices provided along the flexible portion, a subject detecting device that is able to come into contact with the subject, detection means for detecting six degrees of freedom for the position and orientation of the image position and orientation detecting device, the position of each of the plurality of insertion shape detecting devices, and the position or orientation of the subject detecting device and outputting corresponding detection values, and image index creation means for creating image indices indicating the position and orientation of the real-time image of the interior of the subject created by the image creation means, the synthesis means synthesizing the insertion shape, the image indices, and the 3-dimensional image on the basis of the detection values outputted by the detection means to create a 3-dimensional guide image that guides the positions and orientations of the flexible portion and the real-time image with respect to the subject.

The present invention makes it possible to minimally invasively detect the insertion shape of a body cavity probe and the direction of real-time images and to create guide images containing the insertion shape of a body cavity probe and the direction of real-time images.

The objects and profits of the present invention will be further clarified through the following detailed description.

BRIEF DESCRIPTION OF THE DRAWINGS

Fig. 1 is a diagram of the entire configuration of a body cavity probe apparatus in accordance with Embodiment 1 of the present invention;
Fig. 2 is a diagram schematically showing an example of a body surface detecting coil in use;
Fig. 3 is a side view showing a body cavity contact probe;
Fig. 4 is a block diagram showing the configuration of an image processing device;
Fig. 5 is a diagram illustrating reference image data stored in a reference image storage portion;
Fig. 6 is a diagram illustrating a voxel space;
Fig. 7 is a diagram showing an orthogonal basis with an origin set on a transmission antenna in order to indicate position and orientation data;
Fig. 8 is a diagram illustrating, for example, that the center of an ultrasonic tomogram image of the subject is mapped to the voxel space;

Fig. 9 is a diagram illustrating, for example, that body cavity feature points of the subject are mapped to the voxel space;

Fig. 10 is a diagram illustrating that an image index creation circuit creates image index data;

Fig. 11 is a diagram illustrating that an insertion shape creation circuit creates insertion shape data;

Fig. 12 is a diagram showing 3-dimensional human body image data;

Fig. 13 is a diagram illustrating that a synthesis circuit fills image index data and insertion shape data into a voxel space in a synthesis memory;

Fig. 14 is a diagram illustrating 3-dimensional guide image data obtained through observation from the ventral side of the subject;

Fig. 15 is a diagram illustrating 3-dimensional guide image data obtained through observation from the caudal side of the subject;

Fig. 16 is a diagram showing a 3-dimensional guide image and an ultrasonic tomogram image shown on a display device;

Fig. 17 is a flowchart showing the general contents of processing in the present embodiment;

Fig. 18 is a flowchart showing the specific contents of a process of specifying body surface feature points and body cavity feature points on a reference image in Fig. 17;

Fig. 19 is a flowchart showing the specific contents of process of a correction value calculating process in Fig. 17;

Fig. 20 is a diagram illustrating the process in Fig. 19;

Fig. 21 is a flowchart showing the specific contents of a process of creating and displaying ultrasonic tomogram images and 3-dimensional guide images in Fig. 17;

Fig. 22 is a diagram illustrating 3-dimensional image data in Embodiment 2 of the present invention;

Fig. 23 is a diagram illustrating 3-dimensional image data in Embodiment 3 of the present invention;

Fig. 24 is a diagram illustrating 3-dimensional image data in Embodiment 4 of the present invention;

Fig. 25 is a diagram illustrating 3-dimensional image data in Embodiment 5 of the present invention;

Fig. 26 is a block diagram showing the configuration of an image processing device in accordance with Embodiment 6 of the present invention;

Fig. 27 is a diagram illustrating 3-dimensional guide image data generated by a 3-dimensional guide image creation circuit A;

Fig. 28 is a diagram illustrating 3-dimensional guide image data generated by a 3-dimensional guide image creation circuit B; and

Fig. 29 is a diagram showing a 3-dimensional guide image and an optical image shown on the display device.

DETAILED DESCRIPTION OF THE PREFERRED EMBODIMENTS

Embodiments of the present invention will be described with reference to the drawings.

[Embodiment 1]

Embodiment 1 will be described with reference to Figs. I to 21. First, description will be given of the configuration of a body cavity probe apparatus 1 in accordance with Embodiment 1 of the present invention.

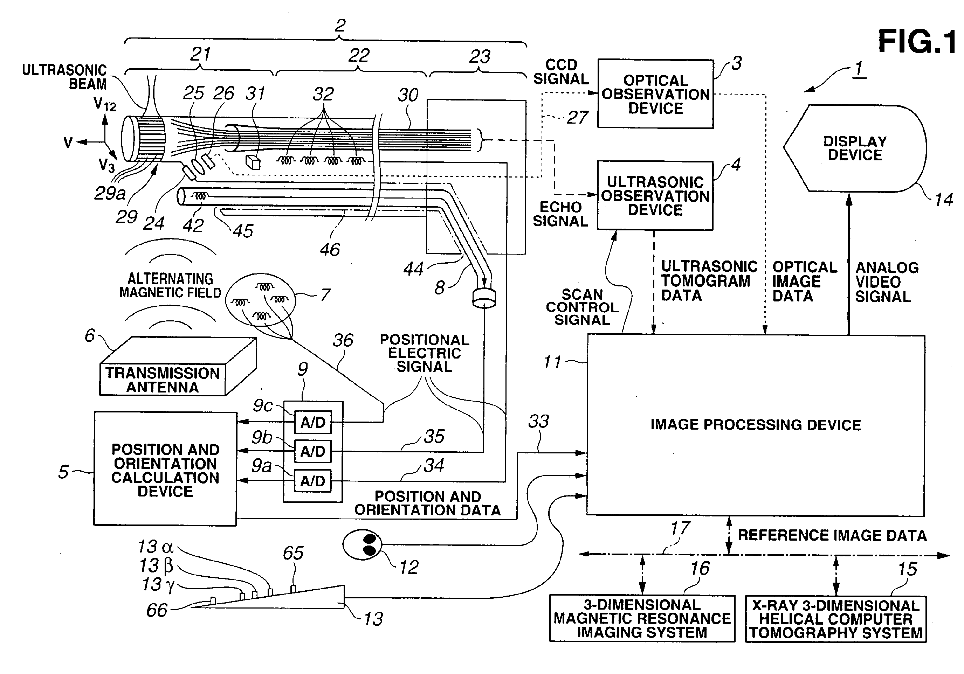

As shown in Fig. 1, the body cavity probe apparatus 1 in Embodiment 1 comprises an electronic radial scanning ultrasonic endoscope 2 as a body cavity probe, an optical observation device 3, an ultrasonic observation device 4, a position and orientation calculation device 5, a transmission antenna 6, a body surface detecting coil 7, a body cavity contact probe 8, an A/D unit portion 9, an image processing device 11, a mouse 12, a keyboard 13, and a display device 14. These components are connected together by signal lines.

An X-ray 3-dimensional helical computer tomography system 15, a 3-dimensional magnetic resonance imaging system 16, and a high-speed network 17 for optical communication or ADSL to which the X-ray 3-dimensional helical computer tomography system 15 and the 3-dimensional magnetic resonance imaging system 16 are connected. The X-ray 3-dimensional helical computer tomography system 15 and the 3-dimensional magnetic resonance imaging system 16 are connected to the image processing device 11 in the body cavity probe apparatus 1 via the network 17.

In order to be inserted into the body cavity such as the esophagus, the stomach, or the duodenum, the ultrasonic endoscope 2 has a rigid potion 21 located at its distal end and composed of a rigid material such as stainless steel, a long-sized flexible portion 22 located closer to the proximal end than the rigid portion 21 and composed of a flexible material, and an operation portion 23 located closer to the proximal end than the flexible portion 22 and composed of a rigid material. The rigid portion 21 and the flexible portion 22 form an insertion portion that is inserted into the body cavity.

The rigid portion 21 has image signal acquisition means fixed thereto to optically pick up images to acquire image signals as described below.

The rigid portion 21 has an optical observation window 24 formed of cover glass. An objective lens 25 and an image pickup device, for example, a CCD (Charge Coupled Device) camera 26, are provided inside the optical observation window 24; the objective lens 25 forms an optical image and the CCD camera 26 is located at the image formation position. Further, an illumination light irradiation window (illumination window; not shown) is provided adjacent to the optical observation window 24 to irradiate the interior of the body cavity with illumination light.

The CCD camera 26 is connected to the optical observation device 3 by a signal line 27. The illumination light irradiation window (not shown) is configured to irradiate illumination light to illuminate the interior of the body cavity. An image of the body cavity surface is formed in the CCD camera 26 through the optical observation window 24 via the objective lens 25. A CCD signal from the CCD camera 26 is outputted, via the signal line 27, to the optical observation device 3, serving as image creation means for generating real-time images of optical images.

The rigid portion 21 also has image signal acquisition means fixed thereto to acoustically perform an image pick-up operation to acquire echo signals as image signals.

The rigid portion 21 has a group of annular arrayed ultrasonic transducers at, for example, a cylindrical distal end thereof; the group of annular arrayed ultrasonic transducers are arranged around the periphery of the insertion shaft and formed by cutting the distal end into pieces like strips of paper. The group of ultrasonic transducers forms an ultrasonic transducer array 29.

Ultrasonic transducers 29a constituting the ultrasonic transducer array 29 are connected to the ultrasonic observation device 4, serving as image creation means for generating ultrasonic real-time images via a corresponding signal line 30 through the operation portion 23. The center of the ring of the ultrasonic transducer array 29 corresponds to the pivoting center of an ultrasonic beam for radial scanning described below.

Here, normal orthogonal bases (unit vectors in the respective directions) V, V3, and V 12 fixed to the rigid portion 21 are defined as shown in Fig. 1.

That is, the vector V is parallel to a longitudinal direction (insertion shaft direction) of the rigid portion 21 and corresponds to a normal vector in an ultrasonic tomogram. The vector $V_3$, which is orthogonal to the vector V, is a three-o'clock direction vector, and the vector $V_{12}$ is a twelve-o'clock direction vector.

In the rigid portion 21, an image position and orientation detecting coil 31 as an image position and orientation detecting device for the ultrasonic transducer array 29 is fixed to a position very close to the center of the ring of the ultrasonic transducer array 29. The image position and orientation detection coil 31 has coils wound in the two directions (axes) of the vectors V and $V_3$ and integrally formed so as to extend in the two axial directions. The image position and orientation detection coil 31 is set to be able to detect both directions of the vectors V and $V_3$.

The flexible portions 22 contains a plurality of insertion shape detecting coils 32 arranged along the insertion shaft, for example, at given intervals to detect the insertion shape of the flexible portion 22 constituting an insertion portion of the ultrasonic endoscope 2.

As shown in Fig. 1, the insertion shape detecting coil 32 is wound in one axial direction and fixed to the interior of the flexible portion 22 so that the winding axial direction aligns with the insertion shaft direction of the flexible portion 22. The rigid portion 21 can be detected on the basis of the position of the image position and orientation detecting coil 31.

Accordingly, to be more exact, the insertion shape detecting device is composed of the image position and orientation detecting coil 31 provided in the rigid portion 21 and the insertion shape detecting coil 32 provided in the flexible portion 22.

The plurality of the insertion shape detecting coils 32 as an insertion shape detecting device for detecting the insertion shape may be provided, for example, only at the distal end of the flexible portion 22 to detect the insertion shape of the distal end of the insertion portion of the ultrasonic endoscope 2.

The present embodiment adopts the plurality of insertion shape detecting coils 32 as an insertion shape detecting device to detect the insertion shape utilizing magnetic fields. This makes it possible to prevent an operator and a patient (subject) from being exposed to radiations in detecting the insertion shape.

A bendable bending portion is often provided in the vicinity of the distal end of the flexible portion 22. The plurality of insertion shape detecting coils 32 may be provided only in the vicinity of the bending portion.

The position and orientation calculation device 5, constituting detection means for detecting the position, orientation, and the like of the image position and orientation detecting coil 31, is connected, via signal lines, to the transmission antenna 6, a plurality of A/D units 9a, 9b, and 9c constituting the A/D unit portion 9, and the image processing device 11, containing insertion shape creation means, 3-dimensional image creation means, synthesis means, image index creation means and the like.

The position and orientation calculation device 5 and the image processing device 11 are connected by, for example, an RS-232C-conforming cable 33.

The transmission antenna 6 is composed of a plurality of transmission coils (not shown) with different winding axis orientations. The transmission coils are integrally housed in, for example, a rectangular housing. The plurality of transmission coils are connected to the position and orientation calculation device 5.

An A/D unit 9i (i = a to c) comprises an amplifier (not shown) that amplifies inputted analog signals and an analog/digital conversion circuit (not shown) that samples the amplified signals and converts the signals into digital data.

The A/D unit 9a is connected individually to the image position and orientation detecting coil 31 and the plurality of insertion shape detecting coils 32 via a signal line 34.

The A/D unit 9b is connected to the elongate body cavity contact probe 8 via a signal line 35. The A/D unit 9c is connected individually to the plurality of body surface detecting coils 7 via a signal line 36.

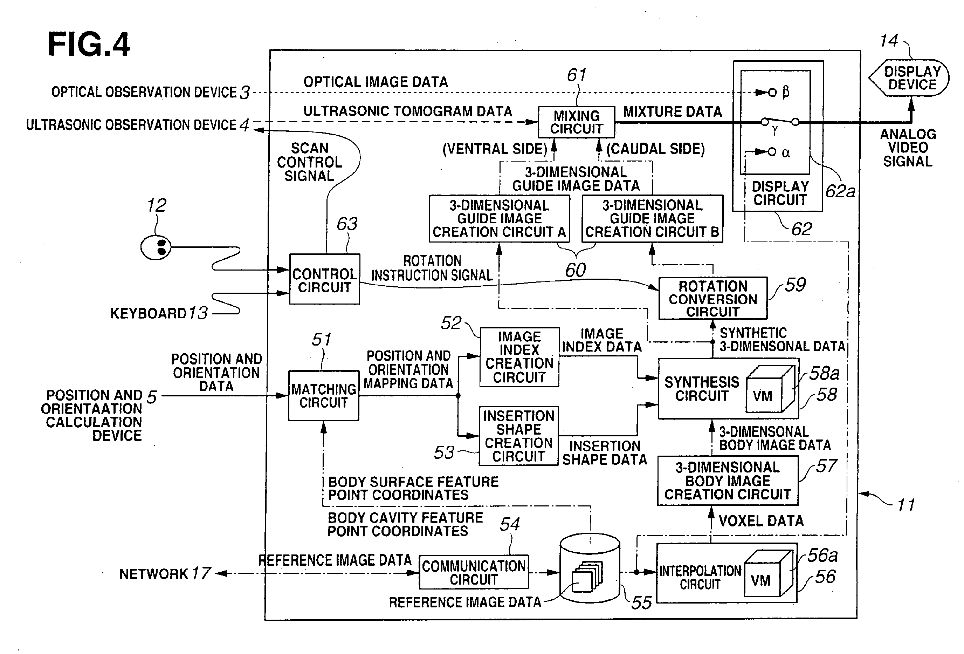

Arrow lines in Fig. 1 and Fig. 4 described below show the flow of signals and data as described below.

(a) First flow: dotted lines indicate the flow of signals and data for optical images.
(b) Second flow: dashed lines indicate the flow of signals and data for ultrasonic tomograms.
(c) Third flow: solid lines indicate the flow of signals and data for positions as well as the flow of data created by processing the signals and data.
(d) Fourth flow: alternate long and short dash lines indicate the flow of reference image data and data created by processing the reference image data.
(e) Fifth flow: thick lines indicate the flow of signals and data for a final display screen obtained by synthesizing ultrasonic tomogram data (described below) with 3-dimensional guide image data (described below).
(f) Sixth flow: curves indicate the flow of signals and data for other control operations.

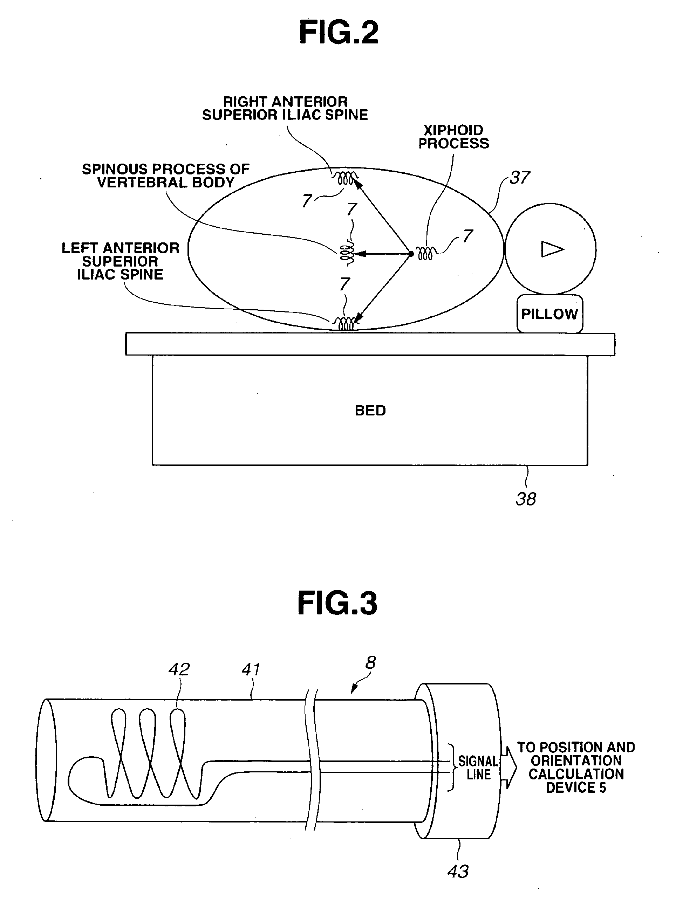

Fig. 2 shows the body surface detecting coil 7, forming a subject detecting device.

The body surface detecting coil 7 comprises four coils wound in one axial direction, respectively, which are each releasably fixed to characteristic points on the body surface of the subject 37, specifically, the surface of the abdomen (these characteristic points are hereinafter simply referred to as body surface feature points) by tapes, belts, bands or the like. The body surface detecting coil 7 is utilized to detect positions using magnetic fields from the body surface feature points.

In normal upper endoscopic inspections, the subject 37 assumes what is called a left lateral position in which the subject 37 lies on his or her left side on a bed 38 and then has the endoscope inserted through his or her mouth. Accordingly, the left lateral position is shown in Fig. 2.

In the description of the present embodiment, the body surface feature points are the "xiphoid process", a characteristic point on the skeleton, the "left anterior superior iliac spine", the left side of the pelvis, the "right anterior superior iliac spine", the right side of the pelvis, and the "spinous process of vertebral body", located on the spine between the right and left anterior superior iliac spine.

The operator can locate the position of the four points through palpation. Further, the four points are not flush with one another but form an un-orthogonal coordinate axis having, as basic vectors, three vectors extending from the xiphoid process as an origin to the respective other feature points. The un-orthogonal coordinate axis is shown in Fig. 2 by thick lines.

Fig. 3 shows the body cavity contact probe 8. The body cavity contact probe 8 has an outer tube 41 composed of a flexible material. A body cavity detecting coil 42 is fixed to the distal end of the interior of the outer tube 41 and has a connector 43 at a proximal end thereof.

As shown in Fig. 3, the body cavity detecting coil 42 is wound in one axial direction and fixed to the distal end of the body cavity contact probe 8. The body cavity detecting coil 42 is fixed so that the winding axis direction thereof aligns with the insertion shaft direction of the body cavity contact probe 8. The body cavity detecting coil 42 is utilized to detect the position of a site of interest or the like in the cavity which is contacted by the distal end of the body cavity contact probe 8.

As shown in Fig. 1, the ultrasonic endoscope 2 comprises a tubular treatment instrument channel 46 including, in the operation portion 23 as a first opening, a treatment instrument insertion port (hereinafter referred to as a forceps port for simplification) 44 through which a pair of forceps or the like is inserted, and a projection port 45 in the rigid portion 21 as a second opening, the tubular treatment instrument channel extending from the operation portion 23 via the flexible portion 22 to the rigid portion 21.

The treatment instrument channel 46 is configured so that the body cavity contact probe 8 can be inserted through the forceps port 44 and project from the projection port 45. The opening direction of the projection port 45 is such that the body cavity contact probe 8 projects from the projection port 45 to fall within the optical visual field range of the optical observation window 24.

Fig. 4 shows the image processing device 11 containing the insertion shape creation means, 3-dimensional image creation means, synthesis means, image index creation means and the like.

The image processing device 11 has a matching circuit 51, an image index creation circuit 52, an insertion shape creation circuit 53, a communication circuit 54, a reference image storage portion 55, an interpolation circuit 56, a 3-dimensional human body image creation circuit 57, a synthesis circuit 58, a rotational transformation circuit 59, and a 3-dimensional image creation circuit 60 (hereinafter referred to as a 3-dimensional guide image creation circuit A and a 3-dimensional guide image creation circuit B) that creates 3-dimensional guide images in two different line-of-sight directions, a mixing circuit 61, a display circuit 62, and a control circuit 63.

Position and orientation data outputted by the position and orientation calculation device 5 is inputted to the matching circuit 51; the position and orientation calculation device 5 constitutes the detection means for detecting the positions and orientations of the insertion shape detecting device and the like.

The matching circuit 51 maps position and orientation data calculated in an orthogonal coordinate axis 0-xyz according to a predetermined conversion equation to calculate new position and orientation data in an orthogonal coordinate axis 0'-x'y'z' as described below.

The matching circuit 51 outputs the new position and orientation data as position and orientation mapping data to the image index creation circuit 52, which creates image index data, and the insertion shape creation circuit 53, which creates insertion shape data.

The communication circuit 54 internally has a high-capacity, high-speed communication modem and is connected to the X-ray 3-dimensional helical computer tomography system 15 which creates 3-dimensional data of human body and the 3-dimensional magnetic resonance imaging system 16 via the network 17.

The reference image storage portion 55 comprises a hard disk drive or the like which can store a large volume of data. The reference image storage portion 55 stores a plurality of reference image data as anatomical image information.

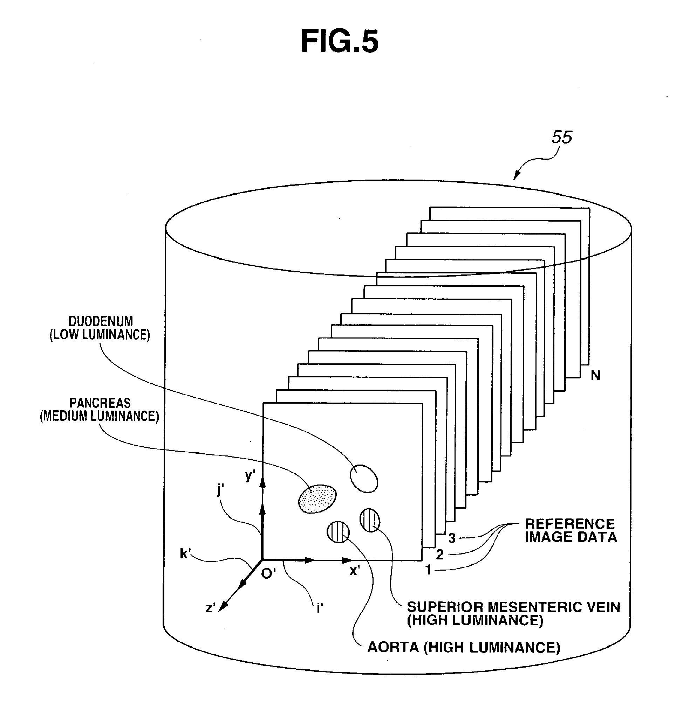

As shown in Fig. 5, reference image data is tomograms of the subject 37 obtained from the X-ray 3-dimensional helical computer tomography system 15 and the 3-dimensional magnetic resonance imaging system 16 via the network 17. In the present embodiment, the reference image data is tomograms shaped like squares with several tens of centimeters on a side which are perpendicular to the body axis (axis extending from the subject's head to feet) and which have a pitch of 0.5 mm to several mm.

In picking up a tomogram of the subject 37, the exposure of the subject 37 to radiations can be reduced or avoided by using the 3-dimensional magnetic resonance imaging system 16 more often than the X-ray 3-dimensional helical computer tomography system 15.

The reference image data in the reference image storage portion 55 in Fig. 5 are denoted by reference numerals 1 to N for convenience of description.

Here, as shown in Fig. 5, an orthogonal coordinate axis 0'-x'y'z' fixed with respect to a plurality of reference image data and normal orthogonal bases therefor (unit vectors in the respective axial directions) i', j', and k' are defined on the reference image data with an origin 0' defined at a lowermost leftmost position of the reference image data no. 1.

As shown in Fig. 4, the interpolation circuit 56 and the synthesis circuit 58 each contain a volume memory VM. For convenience of description, the volume memory VM provided in the interpolation circuit 56 is hereinafter referred to as an interpolation memory 56a. The volume memory provided in the synthesis circuit 58 is hereinafter referred to as a synthesis memory 58a.

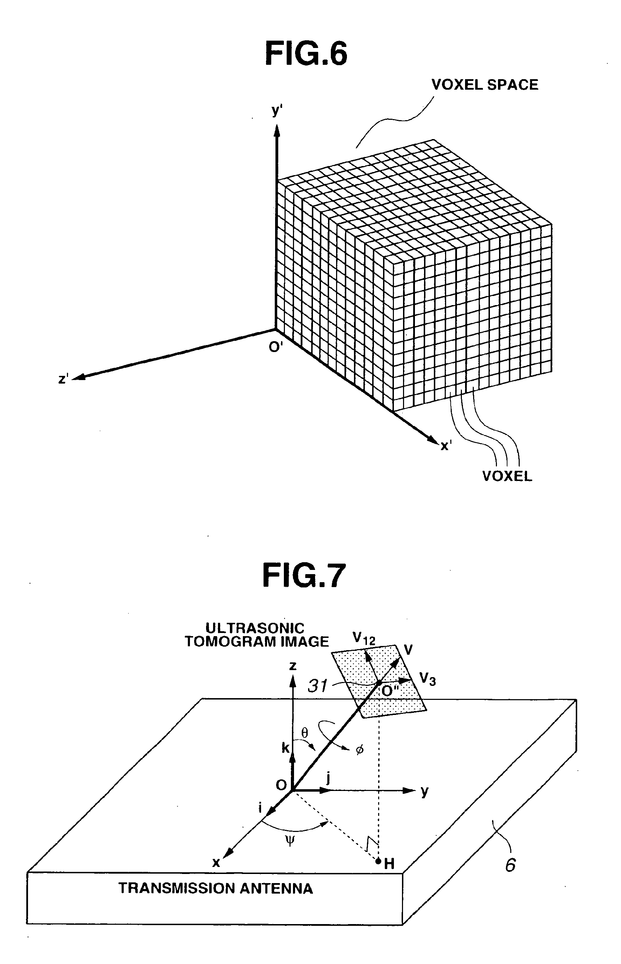

Each of the volume memories VM is configured to be able to store a large volume of data. A voxel space is assigned to a partial storage region of the volume memory VM. As shown in Fig. 6, the voxel space comprises memory cells (hereinafter referred to as voxels) having addresses corresponding to the orthogonal coordinate axis 0'-x'y'z'.

The 3-dimensional human body image creation circuit 57 and the rotational transformation circuit 59, both shown in Fig. 4, each contain a high-speed processor (not shown) that performs high-speed image processing such as extraction by luminance, rotational transformation, similarity transformation, and parallel translation of voxels and pixels; the 3-dimensional human body image creation circuit 57 creates 3-dimensional human body images, and the rotational transformation circuit 59 performs rotational transformation.

The display circuit 62 has a switch 62a that switches inputs to the display circuit 62. The switch 62a has an input terminal $\alpha$, an input terminal $\beta$, an input terminal $\gamma$, and one output terminal. The input terminal $\alpha$ is connected to the reference image storage portion 55. The input terminal P is connected to an output terminal (not shown) of the optical observation device 3. The input terminal $\gamma$ is connected to the mixing circuit 61. The output terminal is connected to the display device 14, which displays optical images, ultrasonic tomograms, and 3-dimensional guide images, and the like.

The control circuit 63 is connected to the portions and circuits in the image processing device 11 via signal lines so as to output instructions to the portions and circuits. The control circuit 63 is connected directly to the ultrasonic observation device 4, a mouse 12, and a keyboard 13 via control lines.

As shown in Fig. 1, the keyboard 13 has a body cavity feature point specification key 65, a scan control key 66, and display switching keys 13$\alpha$, 13$\beta$, and 13$\gamma$.

Depressing any of the display switching keys 13$\alpha$, 13$\beta$, and 13$\gamma$ allows the control circuit 63 to output an instruction to the display circuit 62 to switch the switch 62a to the input terminal $\alpha$, $\beta$, or $\gamma$. Depressing the display switching key 13$\alpha$ allows the switch 62a to be switched to the input terminal $\alpha$. Depressing the display switching key 13$\beta$ allows the switch 62a to be switched to the input terminal $\beta$. Depressing the display switching key 13$\gamma$ allows the switch 62a to be switched to the input terminal $\gamma$.

The signals and data described above in (a) first flow to (f) sixth flow will be sequentially described. (a) The operation of the present embodiment will be described along the first flow of signals and data for an optical image shown by a dotted line.

The illumination light irradiation window (not shown) of the rigid portion 21 irradiates the optical visual field range with illumination light. The CCD camera 26 picks up an image of an object within the optical visual field range and performs a photoelectric conversion to obtain a CCD signal. The CCD camera 26 then outputs the CCD signal to the optical observation device 3.

The optical observation device 3 creates data for a real-time image of the optical visual field range on the basis of the inputted CCD signal. The optical observation device 3 then outputs the data to input terminal β of the switch 62a of the display circuit 62 in the image processing device 11 as optical image data.

(b) The operation of the present embodiment will be described along the second flow of signals and data for an ultrasonic tomogram.

When the operator depresses the scan control key 66, the control circuit 63 outputs a scan control signal to the ultrasonic observation device 4 to instruct a radial scan described below to be controllably turned on and off.

The ultrasonic observation device 4 selects some of the ultrasonic transducers 29a constituting the ultrasonic transducer array 29 and transmits excitation signals shaped like pulse voltages to the selected ultrasonic transducers.

Each of the selected ultrasonic transducers 29a receives and converts the corresponding excitation signal into an ultrasonic wave that is a compressional wave for a medium.

In this case, the ultrasonic observation device 4 delays the excitation signals so that the excitation signals reach the corresponding ultrasonic transducers 29a at different times. The value (delay amount) of the delay is adjusted so that ultrasonic waves excited by the ultrasonic transducers 29a form one ultrasonic beam when allowed to overlap one another in the subject 37.

The ultrasonic beam is emitted to the exterior of the ultrasonic endoscope 2. A reflected wave from the interior of the subject 37 returns to each ultrasonic transducer 29a along a path opposite to that of the ultrasonic beam.

Each ultrasonic transducer 29a converts the reflected wave into an electric echo signal and transmits the signal to the ultrasonic observation device 4 along a path opposite to that of the excitation signal.

The ultrasonic observation device 4 reselects a plurality of the ultrasonic transducers 29a to be involved in the formation of an ultrasonic beam such that the ultrasonic beam pivots in a plane (hereinafter referred to as a radial scan plane) which contains the center of the ring of the ultrasonic transducer array 29 and which is perpendicular to the rigid portion 21 and flexible portion 22. The ultrasonic observation device 4 then transmits excitation signals again to the selected ultrasonic transducers 29a. Thus, the transmission angle of the ultrasonic beam is varied. Repeating this allows what is called a radial scan to be achieved.

In this case, for each radial scan of the ultrasonic transducer array 29, the ultrasonic observation device 4 creates one digitalized ultrasonic tomogram data for a real-time image perpendicular to the insertion shaft of the rigid portion 21 from the echo signals into which the ultrasonic transducers 29a converts the reflected waves. The ultrasonic observation device 4 then outputs the ultrasonic tomogram data to the mixing circuit 61 in the image processing device 11. At this time, the ultrasonic observation device 4 processes the ultrasonic tomogram data into a square.

Thus, in the present embodiment, the ultrasonic observation device 4 reselects a plurality of ultrasonic transducers 29a to be involved in the formation of an ultrasonic beam and transmits excitation signals again. Thus, for example, 12 o'clock direction of a square ultrasonic tomogram is determined depending on which ultrasonic transducer 29a the ultrasonic observation device 4 selects as the 12 o'clock direction in transmitting excitation signals.

Thus, the normal vector V, 3 o'clock vector $V_3$, and 12 o'clock vector $V_{12}$ for the ultrasonic tomogram are defined. The ultrasonic observation device 4 further creates ultrasonic tomogram data obtained through observations from a direction -V opposite to that of the normal vector V.

The following are performed in real time: the radial scan by the ultrasonic transducer array 29, the creation of ultrasonic tomogram data by the ultrasonic observation device 4, and the output to the mixing circuit 61. In the present embodiment, ultrasonic tomograms are generated as real-time images.

(c) Now, the operation of the present embodiment will be described along the third flow of signals and data for positions and of data created by processing the signals and data.

The position and orientation calculation device 5 excites the transmission coil (not shown) in the transmission antenna 6. The transmission antenna 6 generates alternating magnetic fields in a space. The following coils detect and convert alternating magnetic fields into positional electric signals and then output the signals to the A/D units 9a, 9b, and 9c, respectively: two coils constituting the image position and orientation detecting coil 31, which detects the position and orientation (direction) of the image signal acquisition means by ultrasonic waves, the coils wound in the directions of the vectors V and $V_3$ and having orthogonal winding axes, and the plurality of insertion shape detecting coils 32, which detect the insertion shape of the flexible portion 22, as well as the body cavity detecting coil 42 and body surface detecting coil 7, serving as subject detecting devices.

In each of the A/D units 9a, 9b, and 9c, the amplifier amplifies the positional electric signal, and the analog/digital conversion circuit samples and converts the signal into digital data. Each of the A/D units 9a, 9b, and 9c then outputs the digital data to the position and orientation calculation device 5.

Then, on the basis of the digital data from the A/D unit 9a, the position and orientation calculation device 5 calculates the position of the image position and orientation detecting coil 31 and the directions of the orthogonal winding axes thereof, that is, the vectors V and $V_3$. The position and orientation calculation device 5 calculates the outer product $V \times V_3$ of the vectors V and $V_3$, corresponding to the directions of the orthogonal winding axes, and thus the vector $V_{12}$ in the 12 o'clock direction, corresponding to the remaining orthogonal direction is calculated. The position and orientation calculation device 5 thus calculates the orthogonal three directions, that is, the vectors V, $V_3$, and $V_{12}$.

Then, on the basis of the digital data from the A/D units 9a to 9c, the position and orientation calculation device 5 calculates the position of each of the plurality of insertion shape detecting coils 32, the position of each body surface detecting coil 7, and the position of the body cavity detecting coil 42.

The position and orientation calculation device 5 then outputs the position and orientation of the image position and orientation detecting coil 31, the position of each of the plurality of insertion shape detecting coils 32, the position of each of the four body surface detecting coils 7, and the position of the body cavity detecting coil 42 to the matching circuit 51 in the image processing device 11 as position and orientation data.

Now, the position and orientation data will be described below in detail.

As shown in Fig. 7, the present embodiment defines an origin 0 on the transmission antenna 6 and defines the orthogonal coordinate axis 0-xyz and the normal orthogonal bases (unit vectors in the respective axial directions) i, j, and k on an actual space in which the operator inspects the subject 37.

The position of the image position and orientation detecting coil 31 is defined as 0". The image position and orientation detecting coil 31 is fixed to a position very close to the center of the ring of the ultrasonic transducer array 29. Accordingly, the position 0" aligns with the center of radial scanning and with the center of ultrasonic tomograms.

Here, the position and orientation data is defined as follows.

The directional components of a position vector 00" at the position 0" of the image position and orientation detecting coil 31 on the orthogonal coordinate axis 0-xyz:

$(x0, y0, z0)$

The angular components of an Euler angle (described below) indicating the orientation of the image position and orientation detecting coil 31 with respect to the orthogonal coordinate axis 0-xyz:

$(\psi, \theta, \phi)$

The directional components of the position vector of each of the plurality of insertion shape detecting coils 32 on the orthogonal coordinate axis 0-xyz:

$(xi, yi, zi)$ (i denotes a natural number from 1 to the total number of the insertion shape detecting coils 32).

The directional components of the position vectors of the four body surface detecting coils 7 on the orthogonal coordinate axis 0-xyz:

$(xa, ya, za), (xb, yb, zb), (xc, yc, zc), (xd, yd, zd)$

The directional components of the position vector of the body cavity detecting coil 42 on the orthogonal coordinate axis 0-xyz:

$(xp, yp, zp)$

Here, the Euler angle is such that when the orthogonal coordinate axis 0-xyz in Fig. 7 is rotated around the z axis, then around the y axis, and around the z axis again, the directions of the axes align with each other as described below.

i after the rotation = $V_3$, j after the rotation = $V_{12}$, and k after the rotation = V.

$\psi$ denotes the first rotation angle around the z axis, $\theta$ denotes the rotation angle around the y axis, and $\phi$ denotes the second rotation angle around the z axis.

In Fig. 7, H denotes an intersecting point between an xy plane and a perpendicular from the position 0" to the xy plane. The angular components $(\psi, \theta, \phi)$ of the Euler angle correspond to the orientation of the image position and orientation detecting coil 31, that is, the orientation of the ultrasonic tomogram data.

The matching circuit 51 calculates, from the following the first, second, third and fourth data groups, a conversion equation that maps a position and orientation expressed on the orthogonal coordinate axis 0-xyz to a position and orientation in the voxel space expressed on the orthogonal coordinate axis 0'-x'y'z'.

The method for calculation will be described below. The position and orientation data described below for the first and second data groups is varied by movement of the subject 37. New conversion equations are created in conjunction with movement of the subject 37. The creation of a new conversion equation will also be described below.

A first data group included in the position and orientation data includes the directional components (xa, ya, za), (xb, yb, zb), (xc, yc, zc), and (xd, yd, zd) of the position vectors, on the orthogonal coordinate axis 0-xyz, of the body surface detecting coils 7 attached to the xiphoid process, the left anterior superior iliac spine, the right anterior superior iliac spine, and the spinous process of vertebral body of the subject 37.

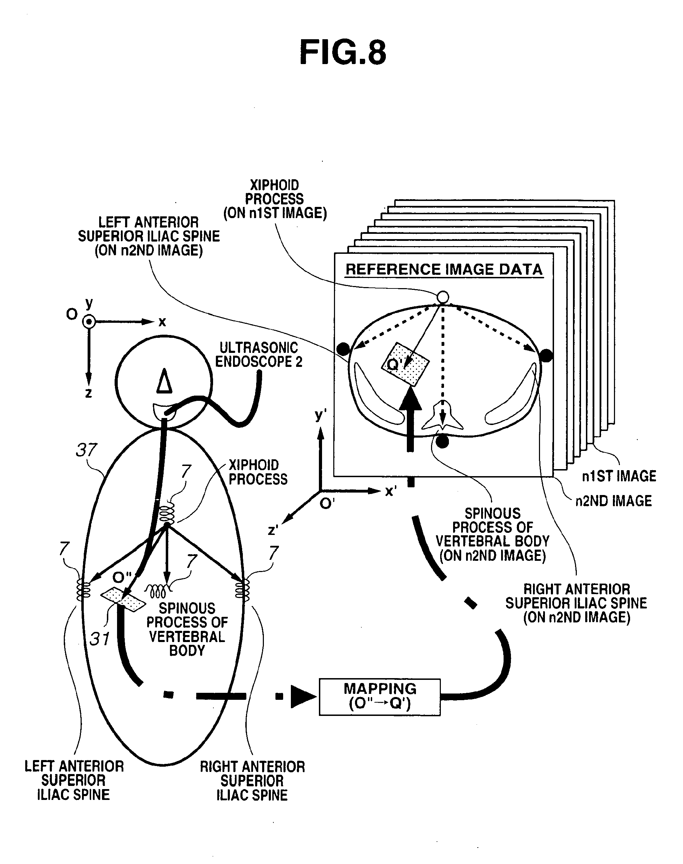

Fig. 8 shows the body surface detecting coils 7 attached to the xiphoid process, the left anterior superior iliac spine, the right anterior superior iliac spine, and the spinous process of vertebral body.

A second data group included in the position and orientation data includes the directional components (xp, yp, zp) of the position vector of the body cavity detecting coil 42 on the orthogonal coordinate axis 0-xyz.

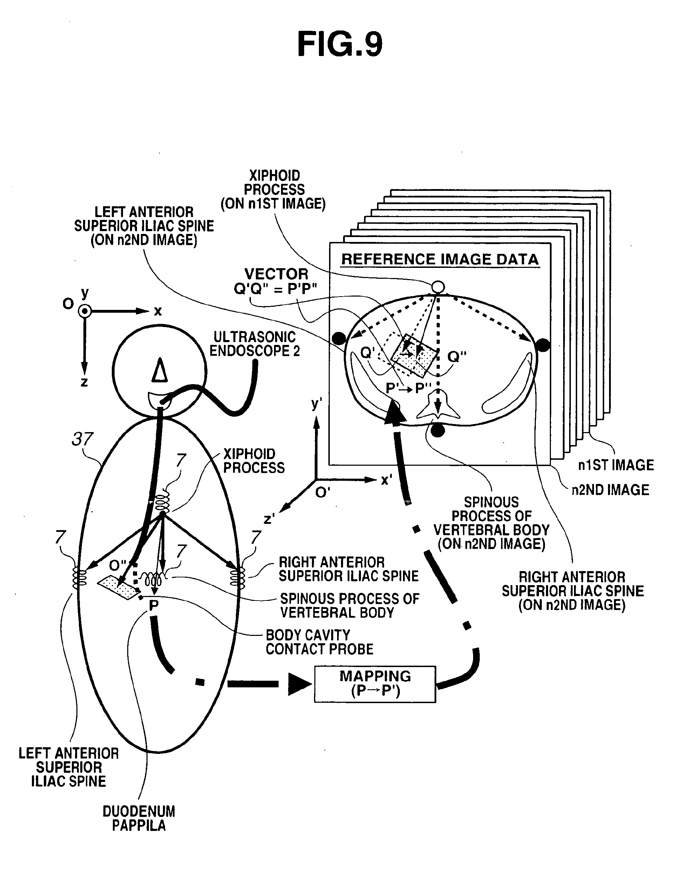

In Fig. 9, a thick dotted line shows the body cavity contact probe 8, containing the body cavity detecting coil 42 fixed to the distal end thereof.

A third data group includes the coordinates (xa', ya', za'), (xb', yb', zb'), (xc', yc', zc'), and (xd', yd', zd'), on the orthogonal coordinate axis 0'-x'y'z', of pixels on any of the reference image data nos. 1 to N which correspond to points on the body surface which are closest to the xiphoid process, the left anterior superior iliac spine, the right anterior superior iliac spine, and the spinous process of vertebral body.

The pixels are pre-specified on any of the reference image data nos. 1 to N by the operator. The method for specification will be described below.

Fig. 9 shows the pixels as black circles ● and white circles ○O. (xa', ya', za'), (xb', yb', zb'), (xc', yc', zc'), and (xd', yd', zd') are read from the reference image storage portion 55 into the matching circuit 51 as body surface feature point coordinates as shown in Fig. 4.

A fourth data group includes the coordinates (xp", yp", zp"), on the orthogonal coordinate axis 0'-x'y'z', of a pixel on any of the reference image data nos. 1 to N which corresponds to the duodenal papilla.

The pixels are pre-specified on any of the reference image data nos. 1 to N by the operator.

The method for specification will be described below. In Fig. 9, the pixel is denoted by P". The coordinate (xp", yp", zp") of the fourth pixel is read from the reference image storage portion 55 into the matching circuit 51 as body cavity feature point coordinates as shown in Fig. 4.

Then, the matching circuit 51 maps the position and orientation data calculated for the orthogonal coordinate axis 0-xyz according to the above conversion equation to calculate new position and orientation data for the orthogonal coordinate axis 0'-x'y'z'.

The matching circuit 51 outputs the new position and orientation data as position and orientation mapping data to the image index creation circuit 52 and the insertion shape creation circuit 53.

The image index creation circuit 52 creates image index data from position and orientation mapping data with a total of six degrees of freedom including the directional components (x0, y0, z0) of the position vector 00", on the orthogonal coordinate axis 0-xyz, of the position 0" of the image position and orientation detecting coil 31 and the angular components ($\psi$, $\theta$, $\phi$) of the Euler angle indicating the orientation of the image position and orientation detecting coil 31 with respect to the orthogonal coordinate axis 0-xyz. The image index creation circuit 52 then outputs the image index data to the synthesis circuit 58.

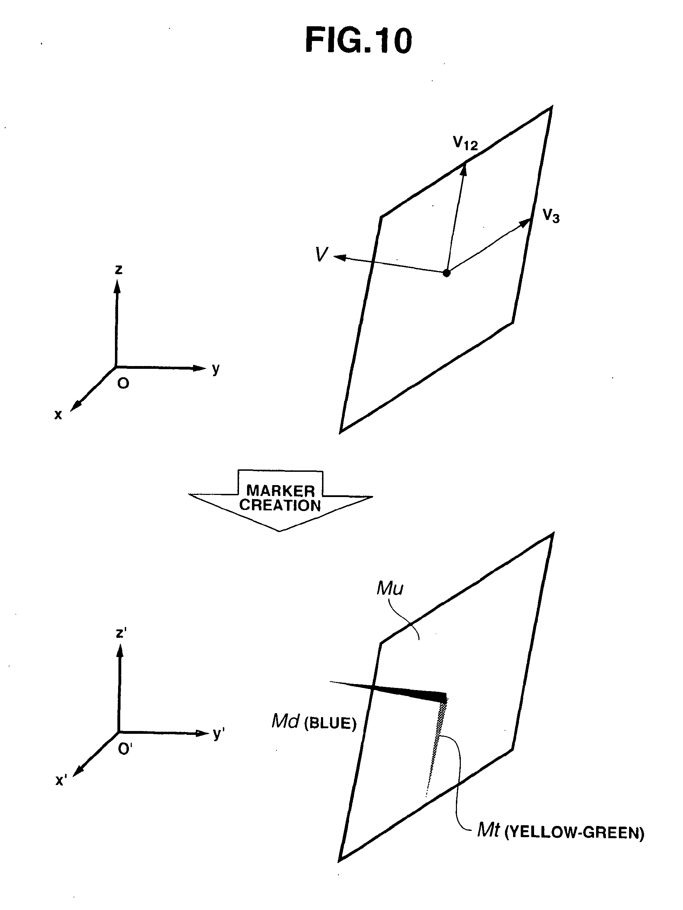

This is shown in Fig. 10. That is, image index data shown in the lower part of Fig. 10 is created from position and orientation mapping data shown in the upper part of Fig. 10.

The image index data is image data on the orthogonal coordinate axis 0'-x'y'z' obtained by synthesizing a parallelogrammatic ultrasonic tomogram marker Mu with, for example, a blue distal direction marker Md (expressed in blue in Fig. 10) and a yellow-green arrow-like 6 o'clock marker Mt (expressed in yellow-green in Fig. 10).

The insertion shape creation circuit 53 creates insertion shape data (through an interpolation and marker creation process) from the position and orientation mapping data including the directional components (x0, y0, z0) of the position vector 00" of the position 0" of the image position and orientation detecting coil 31 and the directional components (xi, yi, zi) of the position vector of each of the plurality of insertion shape detecting coils 32 on the orthogonal coordinate axis 0-xyz. The insertion shape creation circuit 53 then outputs the insertion shape data to the synthesis circuit 58.

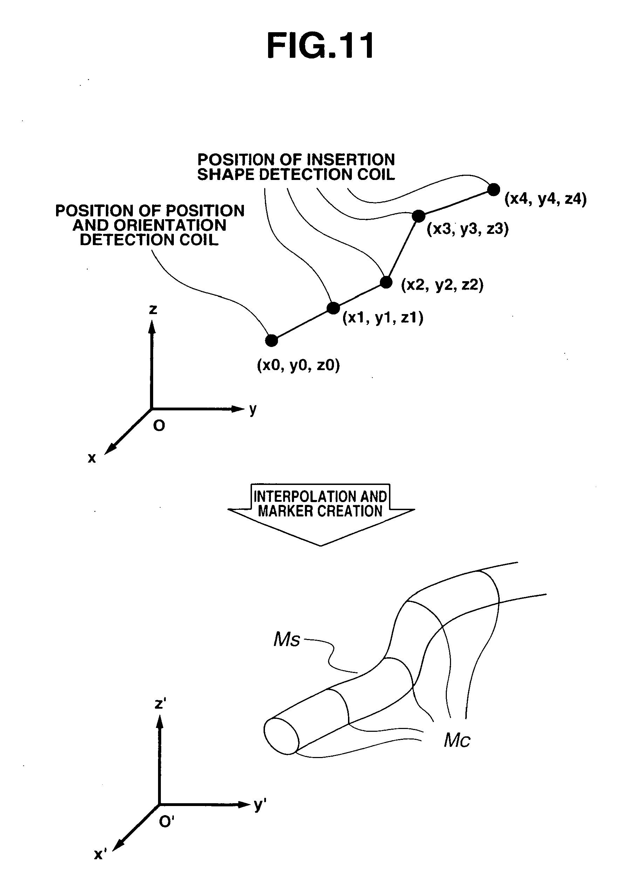

This is shown in Fig. 11. The insertion shape data is image data on the orthogonal coordinate axis 0'-x'y'z' obtained by synthesizing a coil position marker Mc indicating each coil position with a string-like insertion shape marker Ms obtained by sequentially joining together the positions of the image position and orientation detecting coil 31 and the plurality of insertion shape detecting coils 32 and then interpolating the positions.

(d) Now, the operation of the present embodiment will be described along the fourth flow of reference image data and data created by processing the reference image data.

The operator pre-acquires reference image data on the entire abdomen of the subject 37 using the X-ray 3-dimensional helical computer tomography system 15 or the 3-dimensional magnetic resonance imaging system 16.

The operator gives an instruction to acquire reference image data by depressing a predetermined key on the keyboard 13 or selecting from a menu on a screen using the mouse 12. At the same time, the operator indicates from where to acquire the data. In response to the instruction, the control circuit 63 instructs the communication circuit 54 to load the reference image data and indicates to the communication circuit 54 from where to acquire the data.

For example, if the data is to be acquired from the X-ray 3-dimensional helical computer tomography system 15, the communication circuit 54 loads a plurality of two-dimensional CT images through the network 17 as reference image data and stores the images in the reference image storage portion 55.

When the X-ray 3-dimensional helical computer tomography system 15 is used to pick up images, an X ray contrast material is injected through a blood vessel in the subject 37 before image pickup so as to allow blood vessels (in a broad sense, vessels) such as the aorta and the superior mesenteric vein, or an organ containing a large number of blood vessels to be displayed on two-dimensional CT images at a high or medium luminance so as to be differentiated from the surrounding organs having lower luminances.

If for example, the data is to be acquired from the 3-dimensional magnetic resonance imaging system 16, the communication circuit 54 loads a plurality of two-dimensional MRI images through the network 17 as reference image data and stores the images in the reference image storage portion 55.

When the 3-dimensional magnetic resonance imaging system 16 is used to pick up images, an MRI contrast material with a high nuclear magnetic resonance sensitivity is injected through a blood vessel in the subject 37 before image pickup so as to allow blood vessels such as the aorta and the superior mesenteric vein, or an organ containing a large number of blood vessels to be displayed on two-dimensional MRI images at a high or medium luminance so as to be differentiated from the surrounding organs having lower luminances.

Since the operation performed when the operator selects the X-ray 3-dimensional helical computer tomography system 15 as a data source is similar to that performed when the operator selects the 3-dimensional magnetic resonance imaging system 16 as a data source, description will be given only of the operation performed when the X-ray 3-dimensional helical computer tomography system 15 is selected as a data source and when the communication circuit 54 loads a plurality of two-dimensional CT images as reference image data.

Fig. 5 shows an example of reference image data stored in the reference image storage portion 55. Under the effect of the X ray contrast material, the blood vessels such as the aorta and the superior mesenteric vein are displayed at a high luminance, the organ such as the pancreas which contains a large number of peripheral arteries is displayed at a medium luminance, and the duodenum and the like are displayed at a low luminance.

The interpolation circuit 56 reads all the reference image data nos. 1 to N from the reference image storage portion 55. The interpolation circuit 56 sequentially fills the read reference image data into a voxel space in the interpolation memory 56a.

Specifically, the luminances of the pixels in the reference image data are outputted to voxels having addresses corresponding to the pixels. The interpolation circuit 56 then performs interpolation on the basis of the luminance values of the adjacent reference image data to fill empty voxels with the data. In this manner, all the voxels in the voxel space are filled with data (hereinafter referred to as voxel data) based on the reference image data.

The 3-dimensional human body image creation circuit 57 extracts voxels of a high luminance value (mostly indicating the blood vessel) and voxels of a medium luminance value (mostly indicating the organ such as the pancreas which contains a large number of blood vessels) according to the luminance value range from the interpolation circuit 56. The 3-dimensional human body image creation circuit 57 classifies the voxels according to the luminance and colors the voxels.

The 3-dimensional human body image creation circuit 57 then sequentially fills the extracted voxels into a voxel space in the synthesis memory 58a in the synthesis circuit 58 as 3-dimensional human body image data. At this time, the 3-dimensional human body image creation circuit 57 fills the extracted voxels so that the address of each extracted voxel in the voxel space in the interpolation memory 56a is the same as that in the voxel space in the synthesis memory 58a.

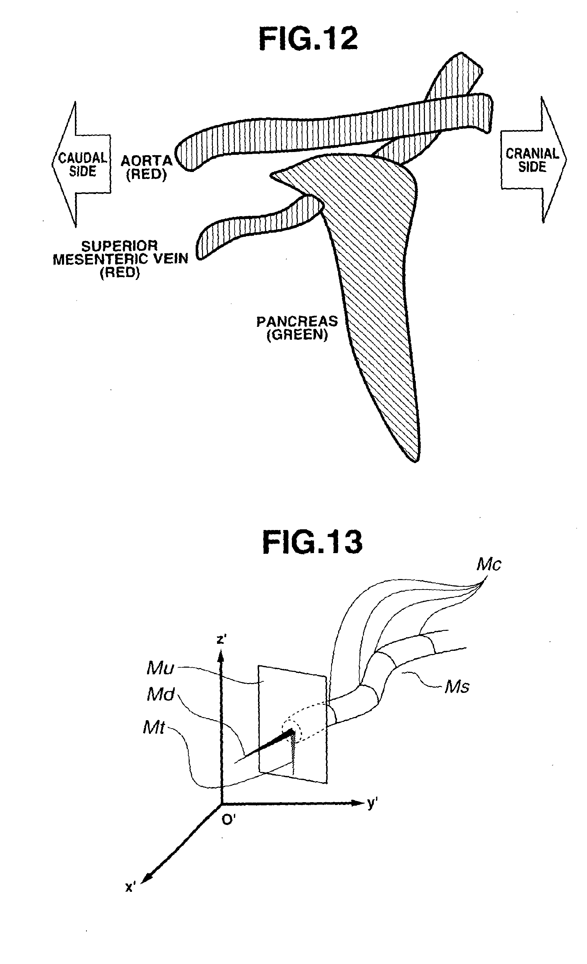

Fig. 12 shows an example of 3-dimensional human body image data. In the example shown in Fig. 12, the 3-dimensional human body image data indicates the blood vessels at a high luminance, the aorta and the superior mesenteric vein, and the organ at a medium luminance, the pancreas. The blood vessels are shown in red, and the pancreas is shown in green. In the 3-dimensional data, the cranial side of the subject 37 corresponds to the right side, and the caudal side of the subject 37 corresponds to the left side, with the subject 37 observed from the ventral side.

The 3-dimensional human body image creation circuit 57 also has the function of extraction means to extract the organ, blood vessels, and the like. The extraction means may be provided in the 3-dimensional guide image creation circuit A or B. Then, when a 3-dimensional guide image is to be created, the 3-dimensional guide image creation circuit A or B may be allowed to select the organ or the blood vessels.

The synthesis circuit 58 sequentially fills image index data and insertion shape data into the voxel space in the synthesis memory 58a. This is shown in Fig. 13.

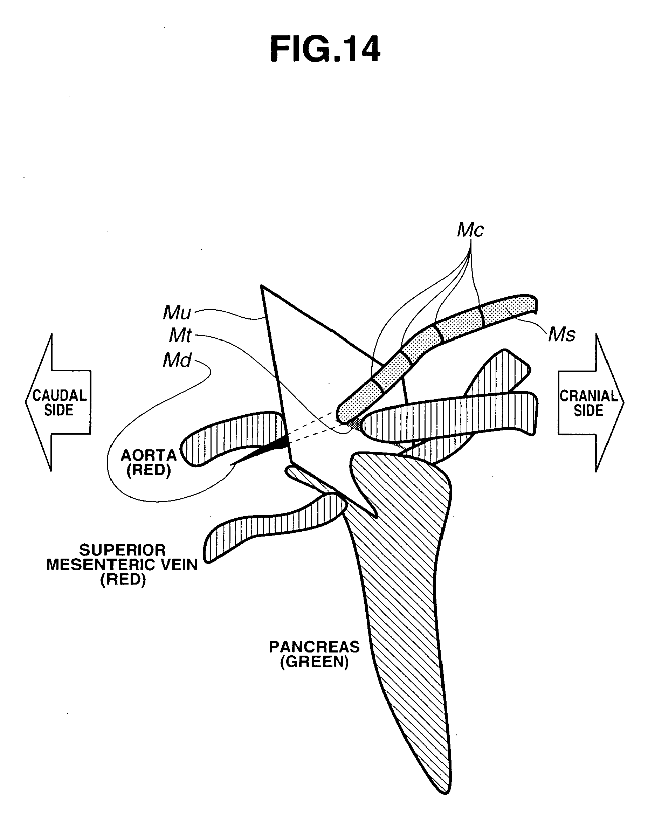

In Fig. 13, for convenience of description, the 3-dimensional human body image data present in the voxel space is omitted (in Fig. 14 and other figures, the 3-dimensional human body image data is not omitted). The synthesis circuit 58 thus fills the 3-dimensional human body image data, the image index data, and the insertion shape data into the same voxel space in the same synthesis memory to synthesize these data into a set of data (hereinafter referred to as synthetic 3-dimensional data).

The rotational transformation circuit 59 reads the synthetic 3-dimensional data and executes a rotating process on the synthetic 3-dimensional data in accordance with a rotation instruction signal from the control circuit 63.

The 3-dimensional guide image creation circuit A executes a rendering process such as hidden surface removal or shading on the synthetic 3-dimensional data to create image data (hereinafter referred to as 3-dimensional guide image data) that can be outputted to the screen.

The default direction of 3-dimensional guide image data is from the ventral side of the body. Accordingly, the 3-dimensional guide image creation circuit A creates 3-dimensional guide image data based on the observation of the subject 37 from the ventral side. Although the default direction of 3-dimensional guide image data is from the ventral side of the body, the 3-dimensional guide image creation circuit A may create 3-dimensional guide image data based on the observation of the subject 37 from the dorsal side. Alternatively, the 3-dimensional guide image creation circuit A may create 3-dimensional guide image data based on the observation from another direction.

The 3-dimensional guide image creation circuit A outputs 3-dimensional guide image data based on the observation from the ventral side of the subject to the mixing circuit 61. The 3-dimensional guide image data is shown in Fig. 14. The right of Fig. 14 corresponds to the subject's cranial side, whereas the left of Fig. 14 corresponds to the subject's caudal side.

In the 3-dimensional guide image data in Fig. 14, the ultrasonic tomogram marker Mu, contained in the image index data, is translucent so that the 6 o'clock direction marker Mt and distal direction marker Md, contained in the image index data, and the insertion shape marker Ms and coil position marker Mc, contained in the insertion shape data, can be seen through.

For the other organs, the ultrasonic tomogram marker Mu is opaque so as to make invisible those parts of the organs which are located behind the ultrasonic tomogram marker Mu. In Fig. 14, markers located behind and overlapping the ultrasonic tomogram marker Mu are shown by dashed lines.

The 3-dimensional guide image creation circuit B executes a rendering process such as hidden surface removal or shading on the rotated synthetic 3-dimensional data to create 3-dimensional guide image data that can be outputted to the screen.

In the present embodiment, by way of example, it is assumed that in response to an input provided by the operator via the mouse 12 and the keyboard 13, the control circuit 63 issues a rotation instruction signal to rotate the 3-dimensional guide image data by 90˚ so that the subject can be observed from the caudal side.

Thus, the 3-dimensional guide image creation circuit B creates 3-dimensional guide image data based on the observation from the caudal side of the subject.

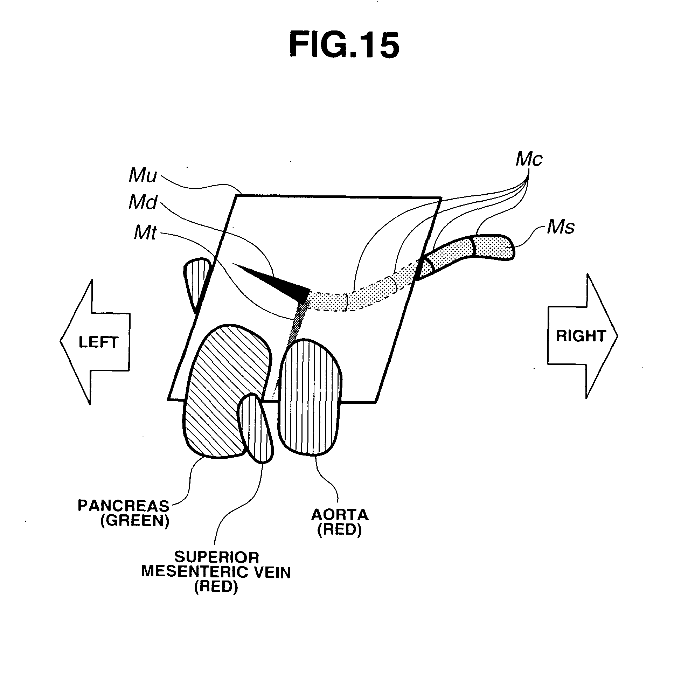

The 3-dimensional guide image creation circuit B outputs 3-dimensional guide image data based on the observation from the caudal side of the subject to the mixing circuit 61. The 3-dimensional guide image data is shown in Fig. 15. The right of Fig. 15 corresponds to the subject's right side, whereas the left of Fig. 15 corresponds to the subject's left side.

In the 3-dimensional guide image data in Fig. 15, the ultrasonic tomogram marker Mu, contained in the image index data, is translucent so that the 6 o'clock direction marker Mt and distal direction marker Md, contained in the image index data, and the insertion shape marker Ms and coil position marker Mc, contained in the insertion shape data, can be seen through.

For the other organs, the ultrasonic tomogram marker Mu is opaque so that the rear side of the ultrasonic tomogram marker Mu cannot be viewed. In Fig. 15, markers located behind and overlapping the ultrasonic tomogram marker Mu are shown by dashed lines.

The ultrasonic tomogram marker Mu shown in Fig. 15 is not in the correct position where the normal of the ultrasonic tomogram marker Mu aligns with an observation line of sight, that is, the normal of the screen of the display device 14. That is, the ultrasonic tomogram marker Mu shown in Fig. 15 is in the incorrect position.

(e) Now, the operation of the present embodiment will be described along the fifth flow of signals and data for a final display screen obtained by synthesizing ultrasonic tomogram data with 3-dimensional guide image data.

The mixing circuit 61 in Fig. 4 creates display mixture data by properly arranging the ultrasonic tomogram data from the ultrasonic observation device 4, the 3-dimensional guide image data from the 3-dimensional guide image creation circuit A based on the observation of the subject 37 from the ventral side, and the 3-dimensional guide image data from the 3-dimensional guide image creation circuit B based on the observation of the subject 37 from the caudal side.

The display circuit 62 converts the mixture data into an analog video signal and outputs the signal to the display device 14.

On the basis of the analog video signal, the display device 14 properly arranges the ultrasonic tomogram, the 3-dimensional guide image based on the observation of the subject 37 from the caudal side, and the 3-dimensional guide image based on the observation of the subject 37 from the ventral side for comparison.

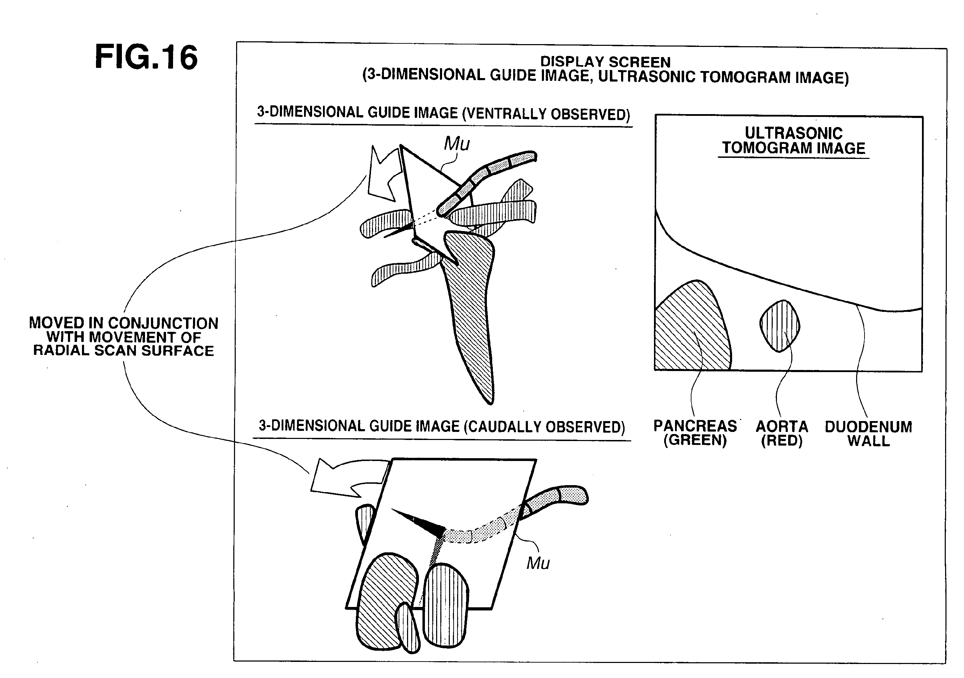

As shown in Fig. 16, the display device 14 displays the organs expressed on the 3-dimensional guide image in the respective colors corresponding to the original luminance values on the reference image data.

In the display example in Fig. 16, the pancreas is displayed in green, and the aorta and the superior mesenteric vein are displayed in red. In Fig. 16, markers located behind and overlapping the ultrasonic tomogram marker Mu are shown by dashed lines.

Further, as shown by white arrows in Fig. 16, the two 3-dimensional guide images move in conjunction with movement of the radial scan surface.

(f) Now, the operation of the present embodiment will be described along the sixth flow of signals and data for control operations.

The following components of the image processing device 11 in Fig. 4 are controlled in accordance with instructions from the control circuit 63: the matching circuit 51, the image index creation circuit 52, the insertion shape creation circuit 53, the communication circuit 54, the reference image storage portion 55, the interpolation circuit 56, the 3-dimensional human body image creation circuit 57, the synthesis circuit 58, the rotational transformation circuit 59, the 3-dimensional guide image creation circuit A, the 3-dimensional guide image creation circuit B, the mixing circuit 61, and the display circuit 62.

The control will be described below in detail.

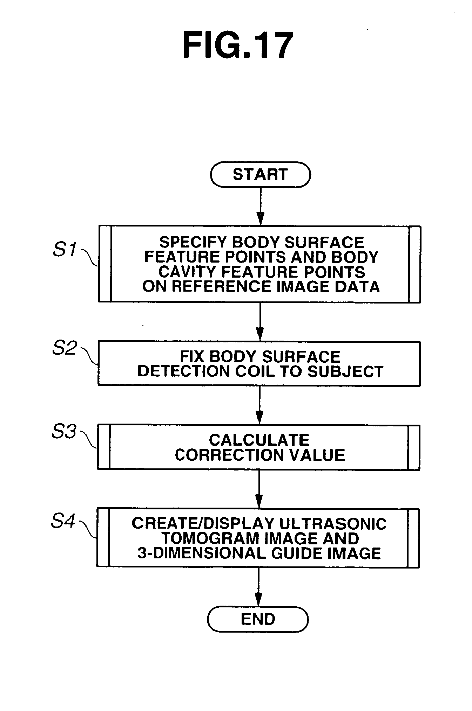

A general description will be given below of how the image processing device 11, the keyboard 13, the mouse 12, and the display device 14 in accordance with the present embodiment work as the operator operates the apparatus. Fig. 17 is a flowchart generally illustrating how the components operate, and the processing in steps S 1 to S4 is executed in the order shown in the figure.

The first step S 1 corresponds to a process of specifying body surface feature points and body cavity feature points on reference image data. That is, in step S1, body surface feature points and body cavity feature points are specified on the reference image data.

In the next step S2, the operator fixes the body surface detecting coil 7 to the subject 37. The operator has the subject 37 lie on his or her left side, that is, has the subject 37 assume what is called a left lateral position. The operator palpates the subject 37 and fixes the body surface detecting coils 7 to positions on the body surface which are closest to the four body surface feature points, the xiphoid process, the left anterior superior iliac spine, the right anterior superior iliac spine, and the spinous process of vertebral body.

The next step S3 corresponds to a process of calculating a correction value.

In step S3, the image processing device 11 acquires position and orientation data on body cavity feature points to calculate a conversion equation that maps position and orientation data expressed on the orthogonal coordinate axis 0-xyz into position and orientation mapping data in the voxel space expressed on the orthogonal coordinate axis 0'-x'y'z'. The image processing device 11 further calculates a correction value for the conversion equation on the basis of the position and orientation data on the body cavity feature points.

The next step S4 executes a process of creating and displaying ultrasonic tomograms and 3-dimensional guide images. In step S4, ultrasonic tomograms and 3-dimensional guide images are created and displayed.

Now, a specific description will be given of the processing in step S1 in Fig. 17, that is, the process of specifying body surface feature points and body cavity feature points on the reference image data.

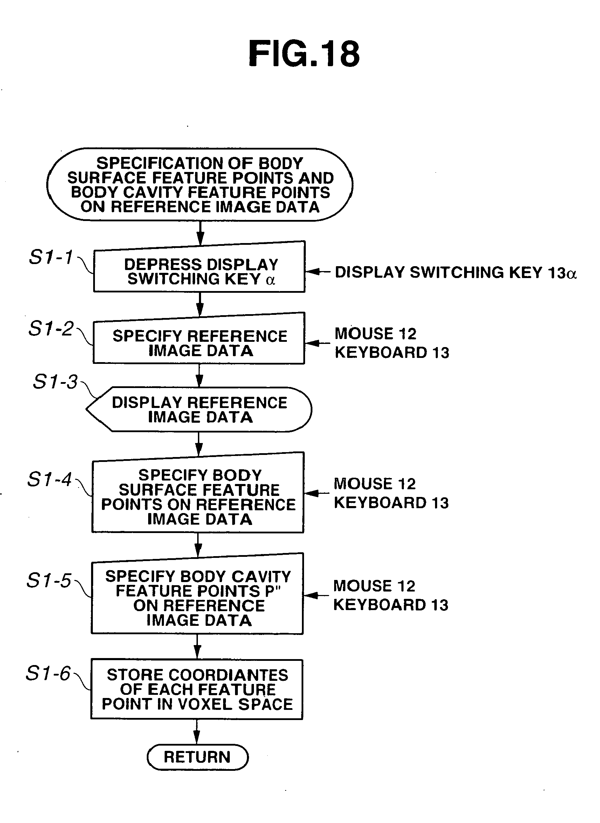

Fig. 18 shows, in detail, the process of specifying body surface feature points and body cavity feature points on the reference image data, which process is executed in step S 1 in Fig. 17.

In the first step S1-1, the operator depresses the display switching key 13α. The control circuit 63 gives an instruction to the display circuit 62. In response to the instruction, the switch 62a in the display circuit 62 is switched to the input terminal α.

In the next step S1-2, the operator uses the mouse 12 and the keyboard 13 to specify any of the reference image data nos. 1 to N.

In the next step S1-3, the control circuit 63 causes the display circuit 62 to read the specified one of the reference image data nos. 1 to N, stored in the reference image storage portion 55.

The display circuit 62 converts the reference image data from the reference image storage portion 55 into an analog video signal, and outputs the reference image data to the display device 14. The display device 14 displays the reference image data.

In the next step S1-4, the operator uses the mouse 12 and the keyboard 13 to specify body surface feature points on the reference image data. Specifically, the operator performs the following operation.

The operator performs an operation such that the displayed reference image data contains any of the four body surface feature points of the subject 37, the xiphoid process, the left anterior superior iliac spine, the right anterior superior iliac spine, and the spinous process of vertebral body. If the reference image data contains none of the feature points, the process returns to step S1-2, where the operator specifies another reference image data. In step S 1-3, the operator repeats displaying a different reference image data until the reference image data contains any of the feature points.

The operator uses the mouse 12 and the keyboard 13 to specify pixels on the displayed reference image data corresponding to points on the body surface of the subject 37 which are closest to the four points on the body surface, the xiphoid process, the left anterior superior iliac spine, the right anterior superior iliac spine, and the spinous process of vertebral body.

The specified points are shown by black circles ● and white circles ○ in Figs. 8 and 9. In the description of the present embodiment, for convenience of description, it is assumed that the xiphoid process O is shown in the reference image data no. n1 (1≤n1≤N) and that the left anterior superior iliac spine, the right anterior superior iliac spine, and the spinous process of vertebral body ● are shown in the reference image data no. n2 (1≤n2≤N).

In Figs. 8 and 9, for convenience of description, the xiphoid process is shown by ○ at the position on the reference image data no. n2 which corresponds to the xiphoid process.

In the step S1-5, the operator uses the mouse 12 and the keyboard 13 to specify a body cavity feature point P". In the present embodiment, by way of example, the body cavity feature point P" is the duodenal papilla (the opening in the common bile duct leading to the duodenum). Specifically, the operator performs the following operation.

The operator uses the mouse 12 and the keyboard 13 to specify any of the reference image data nos. 1 to N.

The control circuit 63 causes the display circuit 62 to read, via a signal line (not shown), the specified one of the reference image data nos. 1 to N, stored in the reference image storage portion 55.

The display circuit 62 outputs the read reference image data to the display device 14. The display device 14 displays the reference image data. If the displayed reference image data does not contain the duodenal papilla, the body cavity feature point of the subject 37, the operator specifies another reference image data. The operator repeats displaying a different reference image data until the displayed reference image data contains the duodenal papilla.

The operator uses the mouse 12 and the keyboard 13 to specify a pixel on the displayed reference image data which corresponds to the duodenal papilla, a point in the body cavity of the subject 37.

The specified point is denoted by P" in Fig. 9. In the description of the present embodiment, for convenience of description, it is assumed the duodenal papilla P" is shown on the reference image data no. n2 (1≤n2≤N).

In the next step S1-6, the control circuit 63 calculates the coordinates, on the orthogonal coordinate axis 0'-x'y'z' in the voxel space, of each of the pixels corresponding to the body surface feature points specified in step S1-4 and of the pixel corresponding to the body cavity feature point P" specified in step S 1-5, on the basis of the addresses on the reference image data. The control circuit 63 then outputs the coordinates to the matching circuit 51.

The calculated value of each of the coordinates, on the orthogonal coordinate axis 0'-x'y'z', of each of the pixels corresponding to the body surface feature points specified in step S1-4 are defined as (xa', ya', za'), (xb', yb', zb'), (xc', yc', zc'), and (xd', yd', zd').

The calculated value of each of the coordinates, on the orthogonal coordinate axis 0'-x'y'z', of the pixel corresponding to the body cavity feature point specified in step S 1-5 is defined as (xp", yp", zp").

The matching circuit 51 stores the coordinates. After step S 1-6, the process proceeds to step S2 in Fig. 17. After the processing in step S2, the process proceeds to the correction value calculation process in step S3 in Fig. 17.

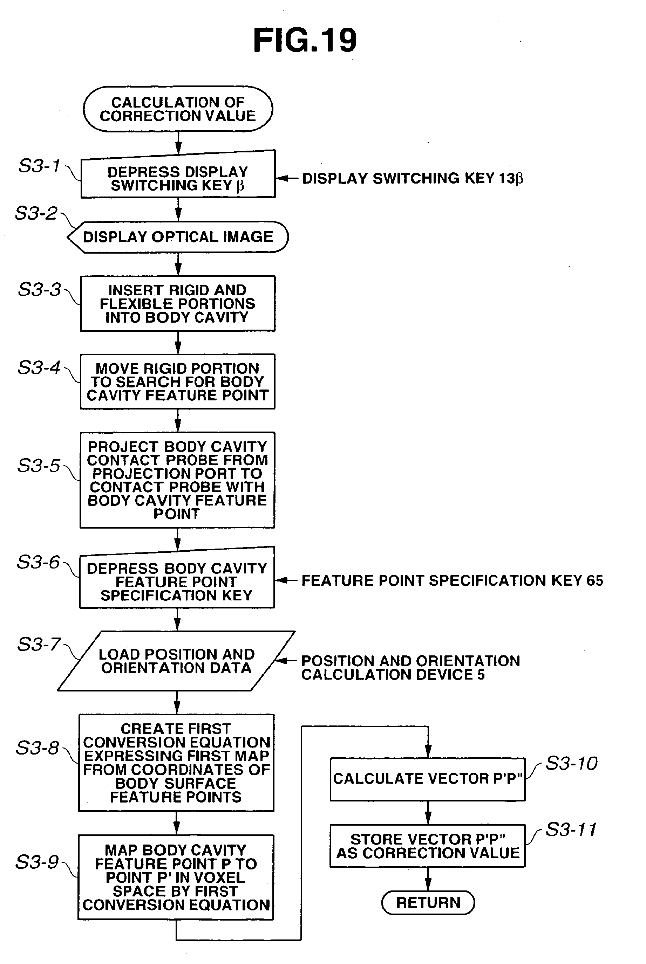

Fig. 19 shows the correction value calculation process in step S3 in detail. As described above, step S3 corresponds to the process of acquiring position and orientation data on the body cavity feature point, calculating a conversion equation that maps position and orientation data expressed on the orthogonal coordinate axis 0-xyz to position and orientation mapping data in the voxel space expressed on the orthogonal coordinate axis 0'-x'y'z', and calculating a correction value for the conversion equation on the basis of the position and orientation data on the body cavity feature point.

When the correction value calculation process in step S3 is started, in the first step S3-1, the operator depresses the display switching key 13β. In response to this instruction, the control circuit 63 gives an instruction to the display circuit 62. The switch 62a in the display circuit 62 is switched to the input terminal β according to the instruction.

In the next step S3-2, the display circuit 62 converts optical image data from the optical observation device 3 into an analog video signal, and outputs the optical image to the display device 14. The display device 14 displays the optical image.

In the next step S3-3, the operator inserts the rigid portion 21 and flexible portion 22 of the ultrasonic endoscope 2 into the body cavity of the subject 37.

In the next step S3-4, while observing the optical image, the operator moves the rigid portion 21 to search for the body cavity feature point. Upon finding the body cavity feature point, the operator moves the rigid portion 21 to the vicinity of the body cavity feature point.

In the next step S3-5, while observing the optical image, the operator inserts the body cavity contact probe 8 through the forceps port 44 and projects the body cavity contact probe 8 from the projection port 45. The operator then brings the distal end of the body cavity contact probe 8 into contact with the body cavity feature point under the optical image field of view.

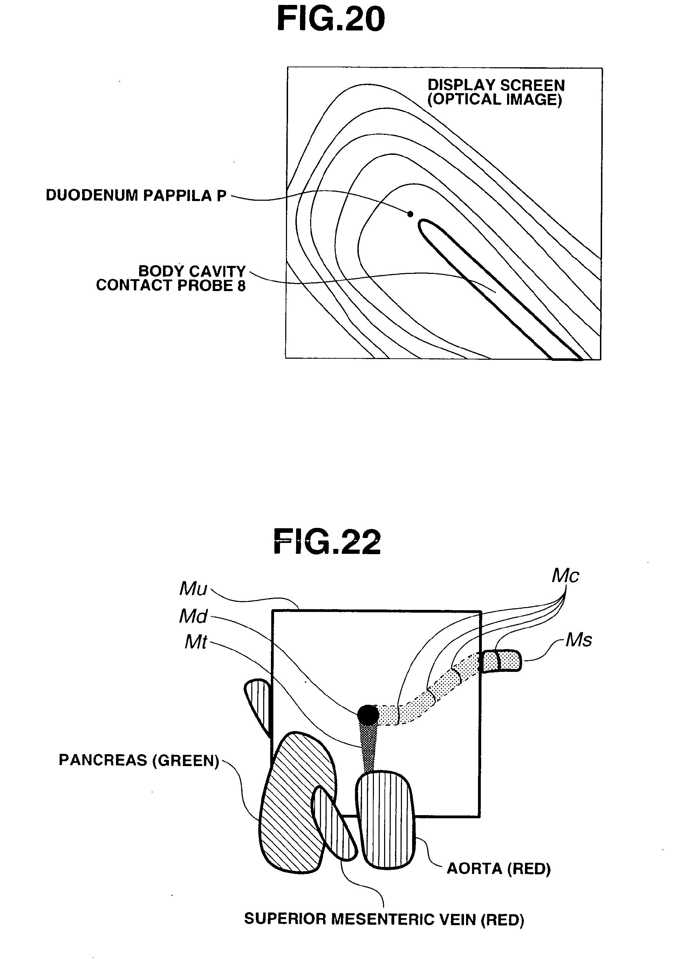

This is shown in Fig. 20. Fig. 20 shows a display screen showing an optical image. The optical image shows the duodenal papilla P as an example of the body cavity feature point, and the body cavity contact probe 8.

In the next step S3-6, the operator depresses the body cavity feature point specification key 65.

In the next step S3-7, the control circuit 63 gives an instruction to the matching circuit 51. In response to the instruction, the matching circuit 51 loads position and orientation data from the position and orientation calculation device 5 and stores the data. The position and orientation data contains the following two types of data as described above.

The directional components of each of the position vectors of the four body surface detecting coils 7 on the orthogonal coordinate axis 0-xyz, that is, in this case, the coordinates of the four body surface feature points on the orthogonal coordinate axis 0-xyz: (xa, ya, za), (xb, yb, zb), (xc, yc, zc), and (xd, yd, zd).

The directional components of the position vector of the body cavity detecting coil 42 on the orthogonal coordinate axis 0-xyz, that is, in this case, the coordinate of the body cavity feature point on the orthogonal coordinate axis 0-xyz: (xp, yp, zp).

In the next step S3-8, the matching circuit 51 creates a first conversion equation expressing a first map, from the coordinates of the body surface feature points. Specifically, this is carried out as follows.

First, the matching circuit 51 already stores the following contents:

First, the coordinates, on the orthogonal coordinate axis 0'-x'y'z' in the voxel space, of the pixels corresponding to the body surface feature points specified in step S1: (xa' ya', za') (xb', yb', zb') (xc', yc', zc') and (xd' yd', zd').

Second, the coordinate, on the orthogonal coordinate axis 0'-x'y'z' in the voxel space, of the pixel corresponding to the body cavity feature point specified in step S1): (xp", yp", zp").

Third, the coordinates, on the orthogonal coordinate axis 0-xyz, of the body surface feature points loaded in step S3-7: (xa, ya, za), (xb, yb, zb), (xc, yc, zc), and (xd, yd, zd).

Fourth, the coordinate, on the orthogonal coordinate axis 0-xyz, of the body cavity feature point loaded in step 3-7: (xp, yp, zp).

The matching circuit 51 creates a first conversion equation that expresses first mapping from any point on the orthogonal coordinate axis 0-xyz to an appropriate point on the orthogonal coordinate axis 0'-x'y'z' in the voxel space, from the third coordinates (xa, ya, za), (xb, yb, zb), (xc, yc, zc), and (xd, yd, zd) and the first coordinates (xa', ya', za'), (xb', yb', zb'), (xc', yc', zc'), and (xd', yd', zd'). The first mapping and the first conversion equation are defined as follows.

As shown in Fig. 8, the xiphoid process, the left anterior superior iliac spine, the right anterior superior iliac spine, and the spinous process of vertebral body, the body surface feature points, are used to assume (set) two nonorthogonal coordinate systems on the subject 37 and in the voxel space (the voxel space is expressed as reference image data in Fig. 8 but is actually a data space obtained by interpolating the reference image data) which use three vectors extending from the xiphoid process to the other points as basic vectors.

The first mapping is mapping from the subject 37 to the voxel space such that the "coordinates of any point on the orthogonal coordinate axis 0-xyz expressed by the nonorthogonal coordinate system on the subject 37" is the same as the "coordinates of a resulting point on the orthogonal coordinate axis 0'-x'y'z' whose coordinates are expressed by the nonorthogonal coordinate system in the voxel space".

Further, the first conversion equation converts the "coordinates of any point on the orthogonal coordinate axis 0-xyz" into the "coordinates, on the orthogonal coordinate axis 0'-x'y'z', of a point in the voxel space resulting from the first mapping".

For example, as shown in Fig. 8, the position of the image position and orientation detecting coil 31, that is, the point of the center of radial scanning and of the center 0" of the ultrasonic tomogram resulting from the first mapping, is defined as Q'.

The coordinates of the point Q' on the orthogonal coordinate axis 0'-x'y'z' are defined as (x0', y0', z0'). The first conversion equation converts the coordinates (x0, y0, z0) of the point 0" on the orthogonal coordinate axis 0-xyz into the coordinates (x0', y0', z0') of the point Q' on the orthogonal coordinate axis 0'-x'y'z'.

In the next step S3-9, the matching circuit 51 maps the body cavity feature point P to the point P' in the voxel space on the basis of the first conversion equation, as shown in Fig. 9. The coordinates of the body cavity feature point P on the orthogonal coordinate axis 0-xyz are (xp, yp, zp). The coordinates of the point P' on the orthogonal coordinate axis 0'-x'y'z' resulting from the first mapping are defined as (xp', yp', zp').

In the next step S3-10, the matching circuit 51 calculates a vector P'P" on the basis of the coordinates (xp', yp', zp') of the point P' on the orthogonal coordinate axis 0'-x'y'z' in the voxel space and the coordinates (xp", yp", zp"), on the orthogonal coordinate axis 0'-x'y'z' in the voxel space, of the point P" corresponding to the body cavity feature point specified in step S1, as follows.

$$P'P'' = (xp'', yp'', zp'') - (xp', yp', zp') = (xp'' - xp', yp'' - yp', zp'' - zp')$$

In the step S3-11, the matching circuit 51 stores the vector P'P". The vector P'P" acts as a correction value used to correct the first conversion equation to create a second conversion equation during a process described below. After step S3-11, the process proceeds to the next step S4.

Now, description will be given of the process of creating and displaying ultrasonic tomograms and 3-dimensional guide images in step S4.

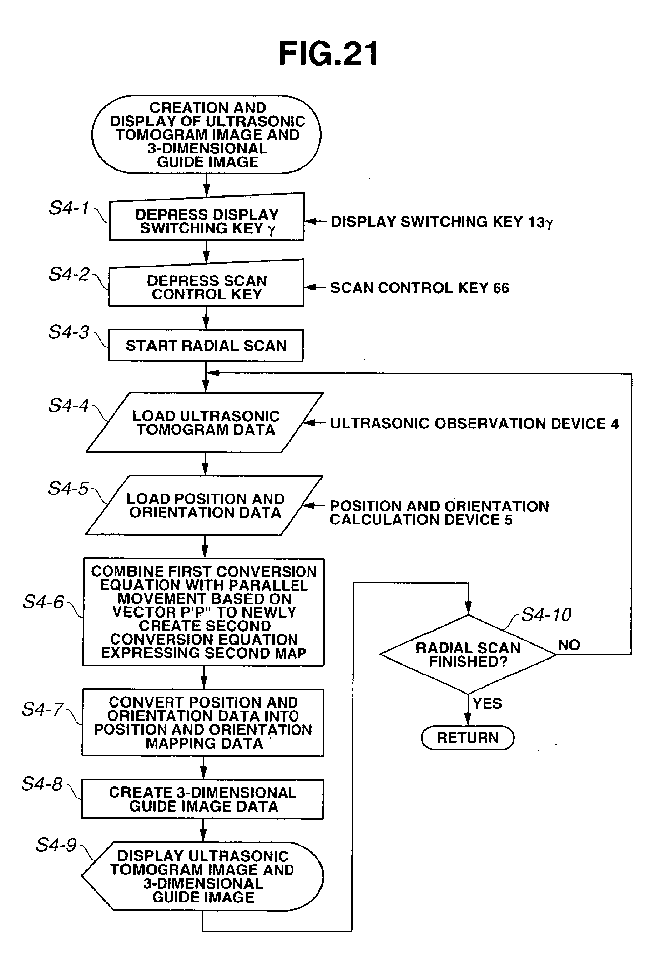

Fig. 21 shows, in detail, the process of creating and displaying actual ultrasonic tomograms and 3-dimensional guide images of the subject 37 in step S4.

When the processing in step S4 is started, in the first step S4-1, the operator depresses the display switching key 13γ. The control circuit 63 gives an instruction to the display circuit 62. The switch 62a in the display circuit 62 is switched to the input terminal γ in response to this instruction.

In the next step S4-2, the operator depresses the scan control key 66.

In the next step S4-3, the control circuit 63 outputs a scan control signal to the ultrasonic observation device 4. Then, the ultrasonic transducer array 29 starts radial scanning.

In the next step S4-4, the control circuit 63 gives an instruction to the mixing circuit 61. In response to the instruction, the mixing circuit 61 sequentially loads ultrasonic tomogram data inputted by the ultrasonic observation device 4 in accordance with the radial scanning.

In the next step S4-5, the control circuit 63 gives an instruction to the matching circuit 51. The matching circuit 51 loads position and orientation data from the position and orientation calculation device 5 and stores the data. The loading is instantaneously performed. Thus, the matching circuit 51 loads the position and orientation data including the following data obtained at the moment when the mixing circuit 61 loads the ultrasonic tomogram data in step S4-4.

The directional components of the position of the image position and orientation detecting coil 31 on the orthogonal coordinate axis 0-xyz, that is, the position vector 00" of the center of radial scanning and of the center 0" of the ultrasonic tomogram:

$(x_0, y_0, z_0)$.

The angular components of the Euler angle indicating the orientation of the image position and orientation detecting coil 31, that is, the orientation of the ultrasonic tomogram, with respect to the orthogonal coordinate axis 0-xyz:

$(\psi, \theta, \phi)$.

The directional components of the position vector of each of the plurality of insertion shape detecting coils 32 on the orthogonal coordinate axis 0-xyz:

$(x_i, y_i, z_i)$ (i is a natural number between 1 and the total number of the insertion shape detecting coils 32).

The direction components of the position vector of each of the four body surface detecting coils 7 on the orthogonal coordinate axis 0-xyz:

$(x_a, y_a, z_a), (x_b, y_b, z_b), (x_c, y_c, z_c), (x_d, y_d, z_d)$.

In the next step S4-6, the matching circuit 51 uses the directional components $(x_a, y_a, z_a), (x_b, y_b, z_b), (x_c, y_c, z_c), (x_d, y_d, z_d)$ of the position vector of each of the four body surface detecting coil 7 on the orthogonal coordinate axis 0-xyz, which are contained in the position and orientation data loaded in step S4-5, to update the first conversion equation stored in step S3).

The matching circuit 51 then combines the updated first conversion equation with the translation of the vector P'P" stored in step S3 to create a new second conversion equation that expresses second mapping.

The matching circuit 51 combines the first conversion equation with the translation of the vector P'P" to create a new second conversion equation that expresses second mapping. The concept of the second mapping is as follows.

$$\text{Second mapping} = \text{first mapping} + \text{translation of the vector P'P"}$$

The translation of the vector P'P" produces a correction effect described below. The vector P'P" acts as a correction value.

The first mapping is mapping from the subject 37 to the voxel space such that the "coordinates of any point on the orthogonal coordinate axis 0-xyz expressed by the nonorthogonal coordinate system on the subject 37" is the same as the "coordinates of a resulting point on the orthogonal coordinate axis 0'-x'y'z' whose coordinates are expressed by the nonorthogonal coordinate system in the voxel space

Ideally, the mapping point P' of the body cavity feature point P created in the voxel space by the first mapping desirably aligns with the point P" corresponding to the body cavity feature point specified in step S1). However, it is actually difficult to accurately align these points with each other.

This is because various factors prevent the "spatial relationship between any point on the orthogonal coordinate axis 0-xyz and the nonorthogonal coordinate system on the subject 37 from completely matching the "spatial positional relationship between a point on the orthogonal coordinate axis 0'-x'y'z' which anatomically corresponds to the above point and the nonorthogonal coordinate system in the voxel space".

This is because, in the case of the present embodiment, although the first mapping and the first conversion equation are determined from the coordinates of the body surface feature points, which are the characteristic points on the skeleton, the duodenal papilla P, which is the body cavity feature point, does not always maintain the same relationship with the body surface feature points on the skeleton.

The main reason is that the X-ray 3-dimensional helical computer tomography system 15 and the 3-dimensional magnetic resonance imaging system 16 normally pick up images of the subject in a supine position, which is different from the left lateral position for inspections with the ultrasonic endoscope 2, thus displacing the organs in the subject 37 under the effect of the gravity.

Thus, the first mapping is combined with the translation of the vector P'P" as a correction value to obtain second mapping. This aligns the mapping point of the body cavity feature point P with the point P" corresponding to the body cavity feature point in the voxel space. Moreover, another point on the subject 37, for example, the center 0" of the ultrasonic tomogram, is also anatomically more accurately aligned with the body cavity feature point by the second mapping.

In the next step S4-7, the matching circuit 51 uses the newly created second conversion equation to convert, into position and orientation mapping data, the directional components (x0, y0, z0) of the position vector 00" of the center 0" of the ultrasonic tomogram on the orthogonal coordinate axis 0-xyz, the angular components ($\psi$, $\theta$, $\phi$) of the Euler angle indicating the orientation of the image position and orientation detecting coil 31 with respect to the orthogonal coordinate axis 0-xyz, and the directional components (xi, yi, zi) (i is a natural number between 1 and the total number of the insertion shape detecting coils 32) of the position vector of each of the plurality of insertion shape detecting coils 32 on the orthogonal coordinate axis 0-xyz, all the directional components being contained in the position and orientation data loaded in step S4-5.

As shown in Fig. 8, the first conversion equation maps the center 0" of the ultrasonic tomogram to the point Q' on the voxel space. However, the second conversion equation newly created in the present step maps the center 0" of the ultrasonic tomogram to the point Q" on the voxel space as shown in Fig. 9. A vector Q'Q" indicating the difference between Q' and Q" coincides with the correction performed by the translation in the second mapping and is thus the same as the vector P'P". That is, the following equation is established.

$$Q'Q" = P'P"$$

In the next step S4-8, the image index creation circuit 52 creates image index data. The insertion shape creation circuit 53 creates insertion shape data.

The synthesis circuit 58 synthesizes 3-dimensional human image data with image index data and insertion shape data to create synthesis 3-dimensional data.

The rotational transformation circuit 59 executes a rotation process on synthetic 3-dimensional data.

Each of the 3-dimensional guide image creation circuits A and B creates 3-dimensional guide image data.

The above processes are as described above.

In the next step S4-9, the mixing circuit 61 properly arranges the ultrasonic tomogram data and the 3-dimensional guide image data to create display mixture data.

The display circuit 62 converts the mixture data into an analog video signal.

On the basis of the analog video signal, the display device 14 properly arranges and displays the ultrasonic tomogram, the 3-dimensional guide image based on the observation of the subject 37 from the ventral side, and the 3-dimensional guide image based on the observation of the subject 37 from the caudal side, as shown in Fig. 16.

The above processes are as described above.

In the next step S4-10, the control circuit 63 determines whether or not the operator depresses the scan control key 66 again, during steps S4-4 to S4-9.

If the operator has depressed the scan control key 66 again, the control circuit 63 ends the above process and outputs a scan control signal to the ultrasonic observation device 4 to instruct the radial scan control to be turned off.

The ultrasonic transducer array 29 ends the radial scan.

If the operator has not depressed the scan control key 66 again, the process jumps to step S4-4.

The processing described in steps S4-4 to S4-9 is thus repeated. Then, the ultrasonic transducer array 29 performs one radial scan, and the ultrasonic observation device 4 creates ultrasonic tomogram data. Every time the ultrasonic observation device 4 inputs ultrasonic tomogram data to the mixing circuit 61, two new 3-dimensional guide images are created and shown on the display screen of the display device 14 together with a new ultrasonic tomogram; the 3-dimensional guide images are properly updated.

That is, as shown in Fig. 16, the ultrasonic tomogram marker Mu, distal direction marker Md, and 6 o'clock direction marker Mt on the image index data and the insertion shape marker Ms and coil position marker Mc on the insertion shape data are moved or deformed on the 3-dimensional human body image data in conjunction with movement of the radial scan surface associated with the operator's manual operation of the flexible portion 22 and the rigid portion 21.

The present embodiment produces the following effects.

According to the present embodiment, the ultrasonic endoscope 2 comprises the rigid portion 21 fixedly having the ultrasonic transducer array 29 that acquires signals for creating ultrasonic tomograms of the interior of the subject 37, the flexible portion 22 located closer to the proximal end than the rigid portion 21, the rigid portion 21 and the flexible portion 22 being provided on the side of the ultrasonic endoscope which is inserted into the body cavity, the ultrasonic observation device 4 that creates ultrasonic tomograms of the interior of the subject 37 from echo signals acquired by the ultrasonic transducers 29a, the image position and orientation detecting coil 31 the position of which is spatially fixed to the rigid portion 21, the plurality of insertion shape detecting coils 32 provided along the flexible portion 22, the plurality of body surface detecting coils 7 that can come into contact with the subject 37, the transmission antenna 6 and the position and orientation calculation device 5 which detect the six degrees of freedom of the position and orientation of the image position and orientation detecting coil 31, the position of each of the plurality of insertion shape detecting coils 32, and the position or orientation of the body surface detecting coil 7 to output position and orientation data, the image index creation circuit 52 that creates the ultrasonic tomogram marker Mu indicating the position and orientation of the ultrasonic tomogram of the interior of the subject 37 created by the ultrasonic observation device 4, the synthesis circuit 58 that synthesizes the insertion shape of the distal end of the flexible portion 22 with the ultrasonic tomogram marker Mu and 3-dimensional human body image data based on the position/orientation data outputted by the position and orientation calculation device 5, and the 3-dimensional guide image creation circuits A and B that guide the positions and orientations of the flexible portion 22 and ultrasonic tomogram with respect to the subject 37.

Thus, the present embodiment can detect the insertion shapes of the rigid portion 21 and flexible portion 22 of the ultrasonic endoscope 2 and the direction of ultrasonic tomograms while minimizing invasive exposure to radiations so as to create the 3-dimensional guide image including both of them.

Further, the present embodiment has the following arrangements and performs the following operations. The image index creation circuit 52 synthesizes the ultrasonic tomogram marker Mu with the blue distal end direction marker Md and the yellow-green arrow-shaped 6 o'clock direction marker Mt to create image index data. The synthesis circuit 58 synthesizes 3-dimensional human body image data, image index data, and insertion shape data in the same voxel space. The mixing circuit 61 creates display mixture data including ultrasonic tomogram data from the ultrasonic observation device 4 and 3-dimensional guide image data which are properly arranged. The display circuit 62 converts the mixture data into an analog video signal. The display device 14 properly arranges the ultrasonic tomograms and 3-dimensional guide images on the basis of the analog video signal.

Thus, the present embodiment can guide the positional relationship between ultrasonic tomograms and an area of interest such as the pancreas. The present embodiment can also guide how the radial scan surface of the ultrasonic endoscope 2, the flexible portion 22, and the rigid portion 21 are oriented and shaped with respect to the body cavity wall such as the digestive tract.

This enables the operator to visually determine these relationships and to perform easily diagnosis, treatment, and the like on the area of interest.

The present embodiment further has the following arrangements and performs the following operations. The matching circuit 51 repeats the processing described in steps S4-4 to S4-9 and further repeats the following process. The matching circuit loads the position and orientation data obtained at the moment when the mixing circuit 61 loads the ultrasonic tomogram data. The matching circuit 51 combines the first conversion equation with the translation of the vector P'P" to newly create a second conversion equation that expresses second mapping. The matching circuit 51 converts, into position and orientation mapping data, the directional components $(x_0, y_0, z_0)$ of the position vector 00" of the center 0" of the ultrasonic tomogram on the orthogonal coordinate axis 0-xyz, the angular components $(\psi, \theta, \phi)$ of the Euler angle indicating the orientation of the image position and orientation detecting coil 31 with respect to the orthogonal coordinate axis 0-xyz, and the directional components $(x_i, y_i, z_i)$ (i is a natural number between 1 and the total number of the insertion shape detecting coils 32) of the position vector of each of the plurality of insertion shape detecting coils 32 on the orthogonal coordinate axis 0-xyz.

The present embodiment thus has the following effect. Even if the posture of the subject 37 changes during inspections with the ultrasonic endoscope 2, unless the positional relationship between the body surface feature points and the organs changes, the ultrasonic tomogram marker Mu, distal marker Md, 6 o'clock direction marker Mt, and insertion shape marker Ms on the 3-dimensional guide image anatomically align with ultrasonic tomogram, flexible portion 22, and rigid portion 21, respectively, more accurately.

The X-ray 3-dimensional helical computer tomography system 15 and the 3-dimensional magnetic resonance imaging system 16 normally pick up images of the subject in the supine position, which is different from the left lateral position for inspections with the ultrasonic endoscope. However, with the arrangements and operations of the present embodiment, the matching circuit 51 combines the first mapping with the translation of the vector P'P'' as a correction value to create the second conversion equation that expresses the second mapping.

Consequently, even if the organs in the subject 37 are displaced under the effect of gravity during ultrasonic endoscopic inspections in the left lateral position, the present embodiment enables more anatomically accurate alignment with a point in the subject 37 by the second mapping, for example, the center 0'' of the ultrasonic tomogram, than the X-ray 3-dimensional helical computer tomography system 15 and the 3-dimensional magnetic resonance imaging system 16. This enables the 3-dimensional guide image to more accurately guide the ultrasonic tomogram.

According to the present embodiment, the arrangements and operations of the 3-dimensional guide image creation circuit A are such that the circuit A creates 3-dimensional image data showing the cranial side in the right of the image and the caudal side in the left of the image and based on the observation of the subject 37 from the ventral side. For ultrasonic endoscopic inspections, the subject 37 is normally inspected in the left lateral position.

The present embodiment also displays 3-dimensional guide images in the left lateral position. This allows the subject 37 to be easily compared with 3-dimensional guide images, while allowing the operator to easily understand the 3-dimensional guide images. The present embodiment therefore can improve or properly support the operator's operations during diagnosis, treatment, or the like.

Further, according to the present embodiment, the 3-dimensional guide image creation circuits A and B create 3-dimensional guide images with the line of sight set in different directions. This enables the positional relationship between the ultrasonic tomogram and the area of interest such as the pancreas to be guided in the plurality of directions and also makes it possible to guide how the ultrasonic tomogram and the flexible portion 22 and rigid portion 21 of the ultrasonic endoscope 2 are oriented and shaped in the plurality of directions with respect to the body cavity wall such as the digestive tract. This makes the operator understand the images easily.

(Variation)

The present embodiment comprises the ultrasonic endoscope 2 including the treatment instrument channel 46 and the body cavity contact probe 8, which is inserted through the treatment instrument channel 46. However, the configuration is not limited to this.

Provided that the objective lens 25 focuses on the body cavity feature point via the optical observation window 24 and the rigid portion 21 itself can be accurately contacted with the body cavity feature point without using the body cavity contact probe 8, the image position and orientation detecting coil 31, fixed to the rigid portion 21, may be used instead of the body cavity detecting coil 42 in the body cavity contact probe 8.

In this case, the image position and orientation detecting coil 31 serves not only as an image position and orientation detecting device but also as a body cavity detecting device.

Furthermore, the present embodiment uses the electronic radial scanning ultrasonic endoscope 2 as an ultrasonic probe. However, it is possible to use a mechanical scanning ultrasonic endoscope such as a body cavity probe apparatus in accordance with the prior art disclosed in Japanese Patent Laid-Open No. 2004-113629, an electronic convex scanning ultrasonic endoscope having a fan-shaped group of ultrasonic transducers provided on one side of the insertion shaft, or a capsule-shaped ultrasonic sonde. The present invention is not limited to the ultrasonic scanning scheme. Alternatively, an ultrasonic probe without the optical observation window 24 may be used.

In the present embodiment, in the rigid portion 21 of the ultrasonic endoscope 2, the ultrasonic transducer is cut into small pieces like strips of paper which are arranged around the periphery of the insertion shaft as an annular array. However, the ultrasonic transducer array 29 may be provided all around the circumference through 360° or may lack in a certain part of the circumference. For example, the ultrasonic transducer 29 may be formed in a part spanning 270° or 180°.

Moreover, with the arrangements and operations of the present embodiment, the transmission antenna 6 and the reception coil are used as position detection means to detect positions and orientations on the basis of magnetic fields. However, the transmission and reception may be reversed. Utilizing magnetic fields to detect the position and orientation enables the formation of position (orientation) detection means of a simple configuration as well as a reduction in costs and sizes.

However, the position (orientation) detection means is not limited to the utilization of magnetic fields. The configuration and operation of the position (orientation) detection means may be such that the position and orientation are detected on the basis of acceleration or another means.

Further, the present embodiment sets the origin 0 at the particular position on the transmission antenna 6. However, the origin 0 may be set in another area having the same positional relationship as that of the transmission antenna 6.

Furthermore, the present embodiment fixes the image position and orientation detecting coil 31 to the rigid portion 21. However, the image position and orientation detecting coil 31 need not be provided inside the rigid portion 21 provided that the position of the image position and orientation detecting coil 31 is fixed with respect to the rigid portion 21.

Moreover, the present embodiment displays the organs on the 3-dimensional guide image data in different colors. However, the present invention is not limited to the use of the different colors (a variation in display color) but may use another aspect using luminance, lightness, chroma saturation, or the like. For example, the different organs may have the respective luminance values.

Further, with the arrangements and operations of the present embodiment, a plurality of two-dimensional CT or MRI images picked up by the X-ray 3-dimensional helical computer tomography system 15 and the 3-dimensional MRI system 16 are used as reference image data. However, it is possible to use 3-dimensional image data pre-acquired using another modality such as PET (Positoron Emission Tomography). Alternatively, it is possible to use 3-dimensional image data pre-acquired using what is called an extracorporeal body cavity probe apparatus, that is, a body cavity probe apparatus which externally applies ultrasonic waves.

Furthermore, with the arrangements and operations of the present embodiment, image data obtained from the subject 37 by the X-ray 3-dimensional helical computer tomography system 15 or the like is used as reference image data. However, it is possible to use image data on another person of the same sex and a similar physique.

Moreover, the present embodiment has the body surface detecting coil 7 comprising the four coils wound in one axial direction and releasably fixed to a plurality of body surface feature points on the subject's body surface using tapes, belts, bands, or the like, to simultaneously obtain position and orientation data on the body surface feature points. However, with the arrangements and operations of the present embodiment, rather than using one coil, for example, the body cavity detecting coil 42, it is possible to lay the subject 37 on the left side before inspections with the ultrasonic endoscope 2 and then to sequentially contact the distal end of the body cavity contact probe 8 with the plurality of body surface feature points to sequentially obtain position and orientation data on the body surface feature points.

Further, according to the present embodiment, the position and orientation calculation means calculated the positions of the body surface detecting coils 7 as position and orientation data. However, instead of the position, the direction of the winding axis may be calculated. Alternatively, both the position and the direction of the winding axis may be calculated. The increased degree of freedom for calculations by the position and orientation calculation device 5 with respect to each body surface detecting coil 7 enables a reduction in the number of body surface detecting coils 7 and thus can reduce the burden imposed on the operator and the subject 37 when the body surface detecting coil 7 is fixed to the subject 37 and during ultrasonic endoscopic inspections.

Furthermore, in the present embodiment, the body surface feature points have been described as the points on the body surface of the abdomen corresponding to the xiphoid process, the left anterior superior iliac spine, the right anterior superior iliac spine, and the spinous process of vertebral body and the body cavity feature point as the duodenal papilla. However, the present invention is not limited to this example. The feature points may be located on the body surface of the chest or in the chest cavity, or any other example may be used. In general, the orientation of the ultrasonic tomogram marker Mu may be more accurately determined when the body surface feature points are taken on these points where they are associated with the skeleton.

Moreover, according to the present embodiment, an input made by the operator via the mouse 12 and the keyboard 13 instructs the control circuit 63 to issue a rotation instruction signal to rotate 3-dimensional guide image data by 90˚, allowing the subject to be observed from the caudal side. The 3-dimensional guide image creation circuit B thus creates 3-dimensional guide image data based on the observation of the subject from the caudal side. However, the present invention is not limited to this example. Alternatively, an input made by the operator via the mouse 12 and the keyboard 13 may allow 3-dimensional guide image to be rotated in real time with respect to the input at any axis or any angle.

[Embodiment 2]

Now, Embodiment 2 of the present invention will be described. The configuration of the present embodiment is the same as that of Embodiment 1. However, the present embodiment is different from Embodiment 1 only in the operation of the 3-dimensional guide image creation circuit B.

Now, the operation of the present embodiment will be described.

As described above, the present embodiment is different from Embodiment 1 only in the operation of the 3-dimensional guide image creation circuit B.

According to Embodiment 1, as shown in Fig. 15, the 3-dimensional guide image creation circuit B created 3-dimensional guide image data based on the observation of the subject from the caudal side and outputted the data to the mixing circuit 61.

Then, the following markers were moved or deformed on the 3-dimensional human body image data in conjunction with movement of the radial scan surface associated with the operator's manual operation of the flexible portion 22 and the rigid portion 21; the ultrasonic tomogram marker Mu, the distal marker Md, and the 6 o'clock direction marker Mt on the image index data as well as the insertion shape marker Ms and the coil position marker Mc on the insertion shape data.

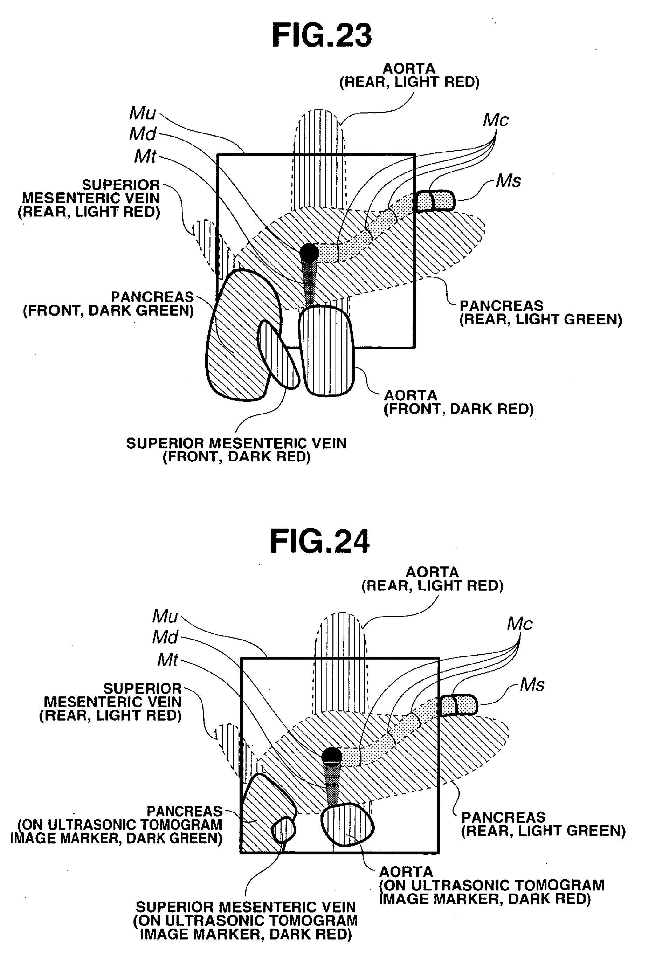

According to the present embodiment, on the basis of the position and orientation mapping data, the 3-dimensional guide image creation circuit B creates guide images with the normal of the ultrasonic tomogram marker Mu set in the correct position with respect to the screen so that the normal coincides with the observation line, that is, the normal of the screen of the display device 14 and with the 6 o'clock direction marker Mt set so as to orient downward on the screen of the display device 14, as shown in Fig. 22.

The 3-dimensional guide image data in Fig. 22 moves on the screen of the display device 14 as the radial scanning surface moves in conjunction with the operator's manual operation of the flexible portion 22 and the rigid portion 21 with the ultrasonic tomogram marker Mu, the distal marker Md, and the 6 o'clock direction marker Mt on the image index data as well as the insertion shape marker Ms and the coil position marker Mc on the insertion shape data all fixed on the screen of the display device 14.

In the 3-dimensional guide image data in Fig. 22, the ultrasonic tomogram marker Mu among the image index data is set to be translucent so that the 6 o'clock direction marker Mt and the distal marker Md on the image index data and the insertion shape marker Ms and the coil position marker Mc on the insertion shape data can be seen through.

For the other organs, the ultrasonic tomogram marker Mu is opaque so as to make invisible those parts of the organs which are located behind the ultrasonic tomogram marker Mu.

The remaining part of the operation is the same as that of Embodiment 1.

The present embodiment produces the following effects.

The arrangements and operations of the present embodiment are such that, on the basis of the position and orientation mapping data, the 3-dimensional guide image creation circuit B creates 3-dimensional guide images with the normal of the ultrasonic tomogram marker Mu set in the correct position with respect to the screen so that the normal coincides with the observation line, that is, the normal of the screen of the display device 14 and with the 6 o'clock direction marker Mt set so as to orient downward on the screen of the display device 14. This allows the direction of the 3-dimensional image to coincide with that of the ultrasonic tomogram placed next to the 3-dimensional guide image and displayed in real time on the screen of the display device 14. Thus, the operator can easily compare these images with each other to anatomically interpret the ultrasonic tomogram.

The other effects of the present embodiment are the same as those of Embodiment 1.

(Variation)

The variation described in Embodiment is applicable as a variation of the present embodiment.

[Embodiment 3]

Now, Embodiment 3 of the present invention will be described.

The configuration of the present embodiment is the same as that of Embodiment 2. The present embodiment is different from Embodiment 2 only in the operation of the 3-dimensional guide image creation circuit B.

Now, the operation of the present embodiment will be described.

As described above, the present embodiment is different from Embodiment 2 only in the operation of the 3-dimensional guide image creation circuit B.

According to Embodiment 2, as shown in Fig. 22, the 3-dimensional guide image creation circuit B created 3-dimensional guide image data by setting the ultrasonic tomogram marker Mu among the image index data to be translucent so that the 6 o'clock direction marker Mt and the distal marker Md on the image index data as well as the insertion shape marker Ms and the coil position marker Mc on the insertion shape data can be seen through, and for the other organs, setting the ultrasonic tomogram marker Mu to be opaque so as to make invisible those parts of the organs which are located behind the ultrasonic tomogram marker Mu. The 3-dimensional guide image creation circuit B then outputted the 3-dimensional guide image data to the mixing circuit 61.

According to the present embodiment, as shown in Fig. 23, the 3-dimensional guide image creation circuit B sets the ultrasonic tomogram marker Mu among the image index data to be translucent. The 3-dimensional guide image creation circuit B creates 3-dimensional image data by allowing not only the 6 o'clock direction marker Mt and distal marker Md on the image index data and the insertion shape marker Ms and coil position marker Mc on the insertion shape data but also those parts of the other organs which are located behind the ultrasonic tomogram marker Mu to be seen through and varying the luminance between the areas in front of and behind the ultrasonic tomogram marker Mu. The 3-dimensional guide image creation circuit B then outputs the data to the mixing circuit 61.

For the pancreas, the area in front of the ultrasonic tomogram marker Mu (the area closer to the operator) is created in dark green, whereas the area behind the ultrasonic tomogram marker Mu is created in light green. For the blood vessel, the area in front of the ultrasonic tomogram marker Mu (the area closer to the operator) is created in dark red, whereas the area behind the ultrasonic tomogram marker Mu is created in light red.

In Fig. 23, markers located behind and overlapping the ultrasonic tomogram marker Mu as well as the organs are shown by dashed lines.

The remaining part of the operation is the same as that of Embodiment 2.

The present embodiment produces the following effects.

The arrangements and operations of the present embodiment are such that the 3-dimensional guide image creation circuit B creates 3-dimensional guide image data by setting the ultrasonic tomogram marker Mu among the image index data to be translucent so that not only the 6 o'clock direction marker Mt and distal marker Md on the image index data and the insertion shape marker Ms and coil position marker Mc on the insertion shape data but also those parts of the other organs which are located behind the ultrasonic tomogram marker Mu can be seen through and varying the luminance between the areas in front of and behind the ultrasonic tomogram marker Mu.

Thus, the operator can easily determine how to further move the flexible portion 22 and the rigid portion 21 in order to display the area of interest such as the diseased part on the ultrasonic tomogram. The operator can thus easily manipulate the flexible portion 22 and rigid portion 21 of the ultrasonic endoscope 2.

In particular, an organ such as the gallbladder which is flexible and mobile inside the subject 37 may not be shown on the ultrasonic tomogram though the organ is shown on the ultrasonic tomogram marker Mu. The 3-dimensional guide image in accordance with the present embodiment may serve as a landmark indicating that the operator can slightly further move the rigid portion 21 and the flexible portion 22 to display the gallbladder on the ultrasonic tomogram. The operator can thus easily manipulate the flexible portion 22 and rigid portion 21 of the ultrasonic endoscope 2.

The other effects are the same as those of Embodiment 1.

(Variation)

The arrangements and operations of the present embodiment are such that the ultrasonic tomogram marker Mu among the image index data set to be translucent so that not only the 6 o'clock direction marker Mt and distal marker Md on the image index data and the insertion shape marker Ms and coil position marker Mc on the insertion shape data but also those parts of the other organs which are located behind the ultrasonic tomogram marker Mu can be seen through. In a variation, the operator may freely vary transparency by providing a selective input via the mouse 12 and the keyboard 13.

The variation of Embodiment 2 is applicable as another variation.

[Embodiment 4]

Now, Embodiment 4 of the present invention will be described. The configuration of the present embodiment is the same as that of Embodiment 3. The present embodiment is different from Embodiment 3 only in the operation of the 3-dimensional guide image creation circuit B.

Now, the operation of the present embodiment will be described.

As described above, the present embodiment is different from Embodiment 3 only in the operation of the 3-dimensional guide image creation circuit B.

According to Embodiment 3, as shown in Fig. 23, the 3-dimensional guide image creation circuit B created 3-dimensional guide image data by setting the ultrasonic tomogram marker Mu among the image index data to be translucent so that not only the 6 o'clock direction marker Mt and distal marker Md on the image index data and the insertion shape marker Ms and coil position marker Mc on the insertion shape data but also those parts of the other organs which are located behind the ultrasonic tomogram marker Mu can be seen through and varying the luminance between the areas in front of and behind the ultrasonic tomogram marker Mu. The 3-dimensional guide image creation circuit B then outputted the data to the mixing circuit 61.

For the pancreas, the area in front of the ultrasonic tomogram marker Mu (the area closer to the operator) was created in dark green, whereas the area behind the ultrasonic tomogram marker Mu was created in light green. For the blood vessel, the area in front of the ultrasonic tomogram marker Mu (the area closer to the operator) was created in dark red, whereas the part behind the ultrasonic tomogram marker Mu was created in light red.

According to the present embodiment, as shown in Fig. 24, the 3-dimensional guide image creation circuit B creates 3-dimensional guide image data by not displaying one of the two areas separated from each other by the ultrasonic tomogram marker Mu among the image index data, that is, the distal end of the flexible portion 22 or the part of the screen of the display device 14 which is closer to the operator, and varying the luminance between the area on the ultrasonic tomogram marker Mu and the area behind the ultrasonic tomogram marker Mu. The 3-dimensional guide image creation circuit B then outputs the 3-dimensional guide image data to the mixing circuit 61.

For the pancreas, the area on the ultrasonic tomogram marker Mu is created in dark green, whereas the area behind the ultrasonic tomogram marker Mu is created in light green. For the blood vessel, the area on the ultrasonic tomogram marker Mu is created in dark red, whereas the area behind the ultrasonic tomogram marker Mu is created in light red.

The remaining part of the operation is the same as that of Embodiment 3.

The present embodiment produces the following effects.

The arrangements and operations of the present embodiment were such that the 3-dimensional guide image creation circuit B created 3-dimensional guide image data by not displaying one of the two areas separated from each other by the ultrasonic tomogram marker Mu among the image index data, that is, the distal end of the flexible portion 22 or the part of the screen of the display device 14 which is closer to the operator, and varying the luminance between the area on the ultrasonic tomogram marker Mu and the area behind the ultrasonic tomogram marker Mu.

Thus, the present embodiment prevents the organs displayed closer to the operator from obstructing the operator's observation of the 3-dimensional guide images. This allows the 3-dimensional guide images to be more easily compared with ultrasonic tomograms displayed, in real time, on the screen of the display device 14 next to the 3-dimensional guide images. This in turn facilitates the anatomical interpretation of the ultrasonic tomograms.

The other effects are the same as those of Embodiment 3.

(Variation)

The variation of Embodiment 3 is applicable as a variation of the present embodiment.

[Embodiment 5]

Now, Embodiment 5 of the present invention will be described. The configuration of the present embodiment is the same as that of Embodiment 1. The present embodiment is different from Embodiment 1 only in the operation of the 3-dimensional guide image creation circuit B.

Now, the operation of the present embodiment will be described.

As described above, the present embodiment is different from Embodiment 1 only in the operation of the 3-dimensional guide image creation circuit B.

According to Embodiment 1, as shown in Fig. 15, the 3-dimensional guide image creation circuit B created 3-dimensional guide image data by setting the ultrasonic tomogram marker Mu among the image index data to be translucent so that the 6 o'clock direction marker Mt and the distal marker Md on the image index data as well as the insertion shape marker Ms and the coil position marker Mc on the insertion shape data can be seen through, and for the other organs, setting the ultrasonic tomogram marker Mu to be opaque so as to make those parts of the organs invisible which are located behind the ultrasonic tomogram marker Mu. The 3-dimensional guide image creation circuit B then outputted the 3-dimensional guide image data to the mixing circuit 61.

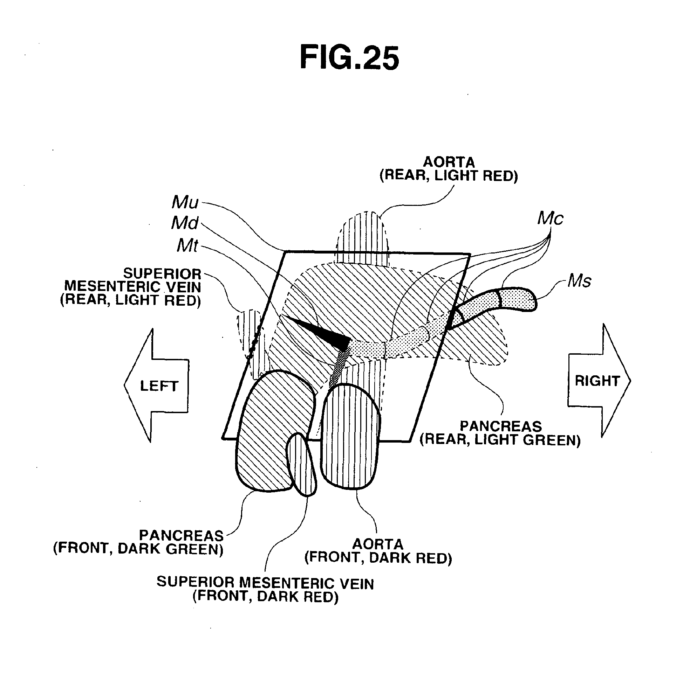

According to the present embodiment, as shown in Fig. 25, the 3-dimensional guide image creation circuit B creates 3-dimensional guide image data by setting the ultrasonic tomogram marker Mu among the image index data to be translucent so that not only the 6 o'clock direction marker Mt and distal marker Md on the image index data and the insertion shape marker Ms and coil position marker Mc on the insertion shape data but also those parts of the other organs which are located behind the ultrasonic tomogram marker Mu can be seen through, and varying the luminance between the area in front of the ultrasonic tomogram marker Mu and the area behind the ultrasonic tomogram marker Mu. The 3-dimensional guide image creation circuit B then outputs the 3-dimensional guide image data to the mixing circuit 61.

For the pancreas, the area which is closer to the distal marker Md than the ultrasonic tomogram marker Mu is created in dark green, whereas the area opposite to the distal marker Md and close to the ultrasonic tomogram marker Mu is created in light green. For the blood vessel, the area which lies closer to the distal marker Md than the ultrasonic tomogram marker Mu is created in dark red, whereas the area opposite to the distal marker Md and close to the ultrasonic tomogram marker Mu is created in light red.

The remaining part of the operation is the same as that of Embodiment 1.

The present embodiment produces the following effects.

The arrangements and operations of the present embodiment were such that the 3-dimensional guide image creation circuit B created 3-dimensional guide image data by setting the ultrasonic tomogram marker Mu among the image index data to be translucent so that not only the 6 o'clock direction marker Mt and distal marker Md on the image index data and the insertion shape marker Ms and coil position marker Mc on the insertion shape data but also those parts of the other organs which are located behind the ultrasonic tomogram marker Mu can be seen through, and varying the luminance between the area in front of the ultrasonic tomogram marker Mu and the area behind the ultrasonic tomogram marker Mu.

Thus, the operator can easily determine how to further move the flexible portion 22 and the rigid portion 21 in order to display the area of interest such as the diseased part on the ultrasonic tomogram. The operator can thus easily manipulate the ultrasonic endoscope 2.

In particular, an organ such as the gallbladder which is flexible and mobile inside the subject 37 may not be shown on the ultrasonic tomogram though the organ is shown on the ultrasonic tomogram marker Mu. The 3-dimensional guide image in accordance with the present embodiment may serve as a landmark indicating that the operator can slightly further move the rigid portion 21 and the flexible portion 22 to display the gallbladder on the ultrasonic tomogram. The operator can thus easily manipulate the ultrasonic endoscope 2.

The other effects are the same as those of Embodiment 1.

(Variation)

The arrangements and operations of the present embodiment were such that the ultrasonic tomogram marker Mu among the image index data was set to be translucent so that not only the 6 o'clock direction marker Mt and distal marker Md on the image index data and the insertion shape marker Ms and coil position marker Mc on the insertion shape data but also those parts of the other organs which were located behind the ultrasonic tomogram marker Mu could be seen through. In a variation, the operator may freely vary transparency via the mouse 12 and the keyboard 13.

The variation of Embodiment 1 is applicable as another variation.

[Embodiment 6]

Now, Embodiment 6 of the present invention will be described. Only differences from Embodiment 1 will be described.

With the image processing device 11 in accordance with Embodiment 1, the rigid portion 21 has the image position and orientation detecting coil 31 fixed to the position very close to the center of the ring of the ultrasonic transducer array 29.

According to the present embodiment, the rigid portion 21 has the image position and orientation detecting coil 31 fixed to a position very close to the CCD camera 26.

The direction in which the image position and orientation detecting coil 31 is fixed is the same as that in accordance with Embodiment 1. The CCD camera 26 has an optical axis which is present in a plane containing V and $V_{12}$ in Fig. 1 and which is directed at a known angle to V.

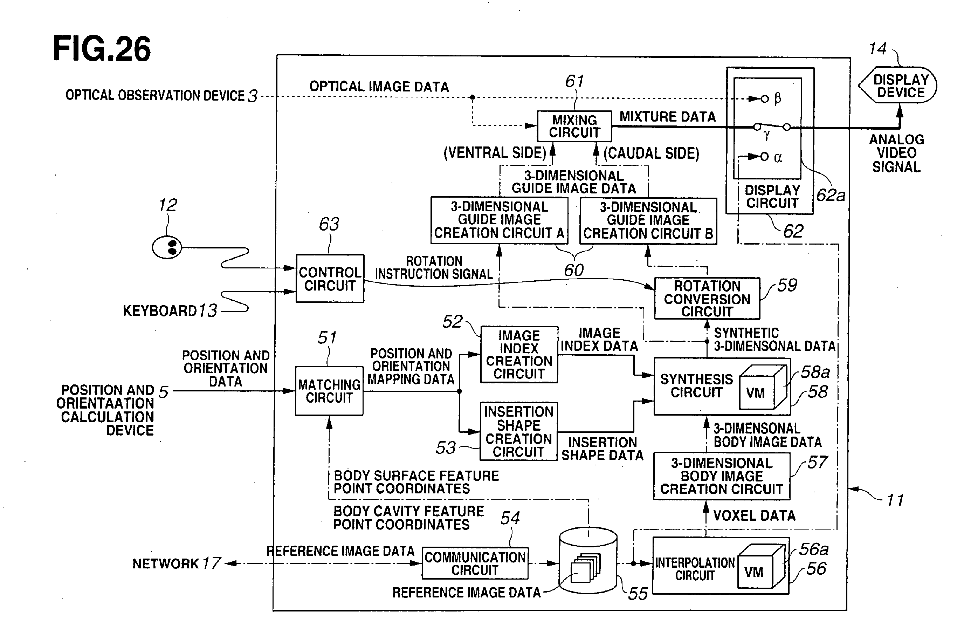

Fig. 26 shows the image processing device 11 in accordance with the present embodiment. In the image processing device 11 in accordance with Embodiment 1, the mixing circuit 61 is connected to the ultrasonic observation device 4. According to the present embodiment, the mixing circuit 61 is connected to the optical observation device 3 in place of the ultrasonic observation device 4.

The other arrangements are the same as those of Embodiment 1.

Now, the operation of the present embodiment will be described.

In the description of the image processing device 11 in accordance with Embodiment 1, the operator selects the X-ray 3-dimensional helical computer tomography system 15 as a data source. The communication circuit 54 loads a plurality of two-dimensional CT images as reference image data. Such reference image data as shown in Fig. 5 are stored in the reference image storage portion 55. For example, under the effect of an X ray contrast material, the blood vessels such as the aorta and superior mesenteric vein are shown at a high luminance. Organs such as the pancreas which contain a large number of peripheral vessels are shown at a medium luminance. The duodenum and the like are shown at a low luminance.

In the present embodiment, description will be given on an example in which the X-ray 3-dimensional helical computer tomography system 15 picks up images of the chest, particularly the trachea, the bronchus, and the carina without contrast and in which, in an area where the bronchus is diverted into two carinas, a carina a and a carina b, the ultrasonic endoscope 2 is inserted into the carina a.

The optical observation device 3 creates optical image data by aligning the 12 o'clock direction (upward direction) of optical images with a direction opposite to the direction in which $V_{12}$ is projected on a plane containing V and $V_{12}$ in Fig. 1.

The 3-dimensional human body image creation circuit 57 extracts voxels with large luminance values (mainly the walls of the trachea, the bronchus, and the carina) from the interpolation circuit 56 and colors the voxels. The 3-dimensional human body image creation circuit 57 then fills the extracted voxels into the voxel space in the synthesis memory 58a of the synthesis circuit 58 as 3-dimensional human body image data.

In this case, the 3-dimensional human body image creation circuit 57 fills the voxels so that the address of the extracted voxel in the voxel space in the interpolation memory 56a is the same as that of the extracted voxel in the voxel space in the synthetic memory. For the 3-dimensional human body image data, the trachea wall, bronchus wall, and carina wall with a high luminance are extracted and colored like the flesh. The subject with his or her head on the right and his or her feet on the left is observed from the ventral side.

The image index creation circuit 52 creates image index data from position and orientation mapping data with a total six degrees of freedom including the directional components (x0, y0, z0) of the position vector 00", on the orthogonal coordinate axis 0-xyz, of the position 0" of the image position and orientation detecting coil 31 and the angular components ($\psi$, $\theta$, $\phi$) of the Euler angle indicating the orientation of the image position and orientation detecting coil 31 with respect to the orthogonal coordinate axis 0-xyz. The image index creation circuit 52 then outputs the image index data to the synthesis circuit 58.

The image index data is image data on the orthogonal coordinate axis 0'-x'y'z' obtained by synthesizing an orange optical-image visual-field direction marker indicating the optical axis with a yellow-green optical-image up direction marker indicating the 12 o'clock direction of optical images.

As is the case with Embodiment 1, the insertion shape creation circuit 53 creates insertion shape data from the position and orientation mapping data including the directional components (x0, y0, z0) of the position vector 00" of the position 0" of the image position and orientation detecting coil 31 and the directional components (xi, yi, zi) of the position vector of each of the plurality of insertion shape detecting coil 32 on the orthogonal coordinate axis 0-xyz. The insertion shape creation circuit 53 then outputs the insertion shape data to the synthesis circuit 58.

This is shown in Fig. 11. The insertion shape data is image data on the orthogonal coordinate axis 0'-x'y'z' obtained by synthesizing the coil position marker Mc indicating each coil position with the string-like insertion shape marker Ms obtained by sequentially joining together the positions of the image position and orientation detecting coil 31 and the plurality of insertion shape detecting coils 32 together and then interpolating the positions.

The synthesis circuit 58 sequentially fills image index data and insertion shape data into the voxel space in the synthesis memory 58a. The synthesis circuit 58 thus sequentially fills the 3-dimensional human body image data, the image index data, and the insertion shape data into the same voxel space in the same synthesis memory 58a to synthesize these data into a set of synthetic 3-dimensional data.

The rotational transformation circuit 59 reads the synthetic 3-dimensional data and executes a rotating process on the synthetic 3-dimensional data in accordance with a rotation instruction signal from the control circuit 63.

The 3-dimensional guide image creation circuit A executes a rendering process such as hidden surface removal or shading on the synthetic 3-dimensional data to create 3-dimensional guide image data that can be outputted to the screen. The default direction of 3-dimensional guide image data is from the ventral side of human body.

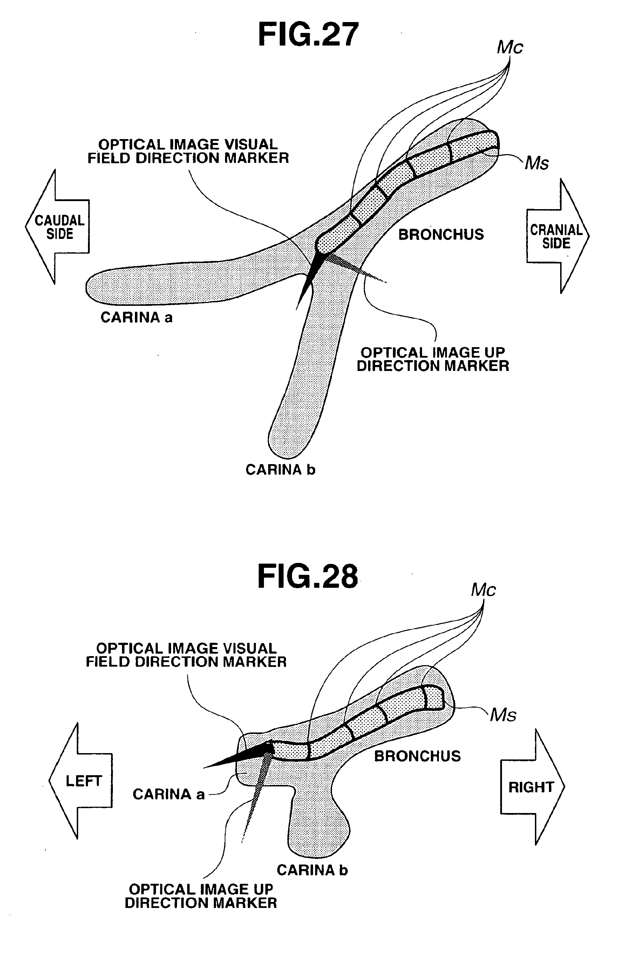

Accordingly, the 3-dimensional guide image creation circuit A creates 3-dimensional guide image data based on the observation of the subject 37 from the ventral side. The 3-dimensional guide image creation circuit A outputs 3-dimensional guide image data based on the observation from the ventral side of the subject to the mixing circuit 61. The 3-dimensional guide image data is shown in Fig. 27. The right of Fig. 27 corresponds to the subject's cranial side, whereas the left of Fig. 27 corresponds to the subject's caudal side.

In the 3-dimensional guide image data in Fig. 27, the wall of the bronchus and the walls of the carinas a and b located beyond the bronchus are translucent so that the optical-image visual-field direction marker and optical-image up direction marker on the image index data and the insertion shape marker Ms and coil position marker Mc on the insertion shape data are visible.

The 3-dimensional guide image creation circuit B executes a rendering process such as hidden surface removal or shading on the synthetic 3-dimensional data subjected to a rotating process to create 3-dimensional guide image data that can be outputted to the screen.

In the present embodiment, by way of example, it is assumed that an input provided by the operator via the mouse 12 and the keyboard 13 instructs the control circuit 63 to issue a rotation instruction signal to rotate the 3-dimensional guide image data through 90° so that the subject can be observed from the caudal side.

Accordingly, the 3-dimensional guide image creation circuit B creates 3-dimensional guide image data based on the observation from the caudal side of the subject. The 3-dimensional guide image creation circuit B outputs 3-dimensional guide image data based on the observation from the caudal side of the subject to the mixing circuit 61. The 3-dimensional guide image data is shown in Fig. 28. The right of Fig. 28 corresponds to the subject's right side, whereas the left of Fig. 28 corresponds to the subject's left side.

In the 3-dimensional guide image data in Fig. 28, the wall of the bronchus and the walls of the carinas a and b located beyond the bronchus are translucent so that the optical-image visual-field direction marker and optical-image up direction marker on the image index data and the insertion shape marker Ms and coil position marker Mc on the insertion shape data are visible.

The mixing circuit 61 creates display mixture data by properly arranging the optical image data from the optical image observation device 3, the 3-dimensional guide image data from the 3-dimensional guide image creation circuit A based on the observation of the subject 37 from the ventral side, and the 3-dimensional guide image data from the 3-dimensional guide image creation circuit B based on the observation of the subject 37 from the caudal side.

The display circuit 62 converts the mixture data into an analog video signal.

On the basis of the analog video signal, the display device 14 properly arranges the optical image, the 3-dimensional guide image based on the observation of the subject 37 from the caudal side, and the 3-dimensional guide image based on the observation of the subject 37 from the ventral side for display.

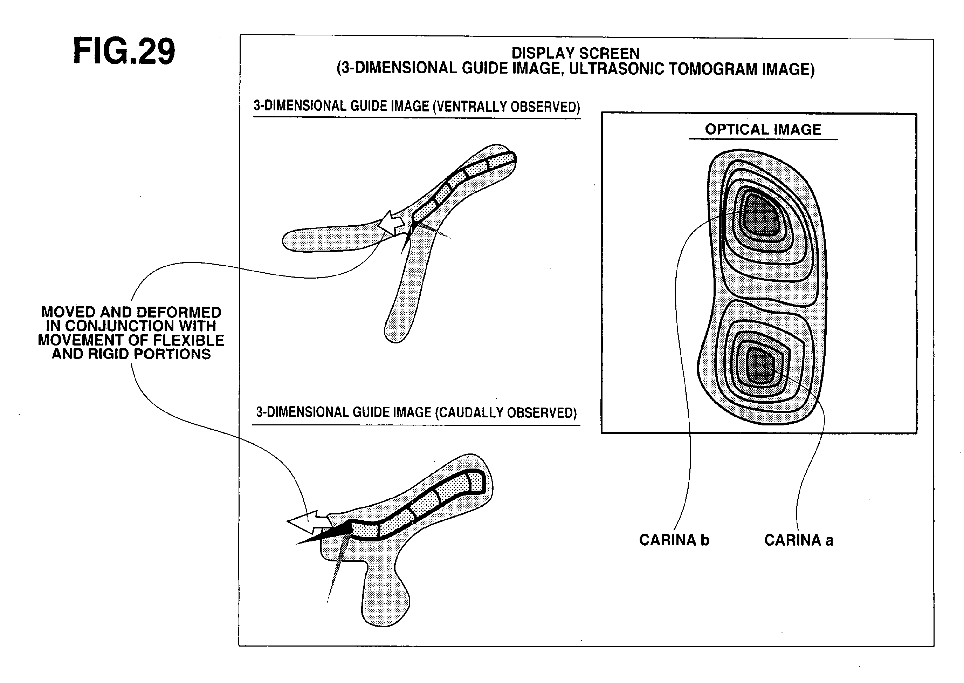

As shown in Fig. 29, the display device 14 displays the walls of the bronchus and carinas expressed on the 3-dimensional guide image in a flesh color.

In the present embodiment, optical images are processed as real-time images.

Like Embodiment 1, the present embodiment creates and displays two new 3-dimensional guide images on the display screen of the display device 14 together with a new optical image while updating the images in real time. That is, as shown in Fig. 29, the optical-image visual-field direction marker and optical-image up direction marker on the image index data and the insertion shape marker Ms and coil position marker Mc on the insertion shape data are moved or deformed on the 3-dimensional human body image data in conjunction with movement of the optical axis associated with the operator's manual operation of flexible portion 22 and the rigid portion 21.

The remaining part of the operation is the same as that of Embodiment 1.

The present embodiment provides the following effects.

The arrangements and operations of the present embodiment are such that the 3-dimensional guide image data is created so that the wall of the bronchus and the walls of the carinas a and b located beyond the bronchus are translucent so that the optical-image visual-field direction marker and optical-image up direction marker on the image index data and the insertion shape marker Ms and coil position marker Mc on the insertion shape data are visible and such that the mixing circuit 61 and the display device 14 properly arrange the optical image, the 3-dimensional guide image based on the observation of the subject 37 from the ventral side, and the 3-dimensional guide image based on the observation of the subject 37 from the caudal side for display.

Thus, the present embodiment can prevent the operator from inadvertently inserting the ultrasonic endoscope 2 (or an endoscope as described in the variation described below) into the carina b instead of the carina a.

The other effects are the same as those of Embodiment 1.

In the above description, the ultrasonic endoscope is inserted into the deep side of the bronchus. However, in other cases, the operator can also insert the body cavity probe into the body cavity to perform smooth diagnosis and treatment because 3-dimensional guide image data is created so that the optical-image visual-field direction marker and optical-image up direction marker on the image index data and the insertion shape marker Ms and coil position marker Mc on the insertion shape data are visible. Thus, a body cavity probe is realized with which the operator can smoothly perform diagnosis and treatment.

(Variation)

Like Embodiment 1, the present embodiment uses the electronic radial scanning ultrasonic endoscope 2 having the optical observation system (the optical observation window 24, objective lens 25, the CCD camera 26, and the illumination light irradiation window (not shown)) serving as a body cavity probe as in the case of Embodiment 1. However, the body cavity probe may be an endoscope simply having an optical observation system in place of the ultrasonic endoscope 2.

The variation of Embodiment 1 is applicable as another variation.

For example, embodiments into which the above embodiments and the like are partly combined also belong to the present invention. Further, the block configuration of the image processing device 11 shown in Fig. 4 and other figures may be changed.

Moreover, the present invention is not limited to the above embodiments. Of course, many variations and applications may be made to the embodiments without departing from the spirit of the present invention.

Obviously, according to the present invention, significantly different embodiments can be constructed on the basis of the present invention without departing from the spirit and scope of the present invention. The present invention is not limited by any particular embodiment thereof but only by the accompanying claims.

**Claims**

**1.** A body cavity probe apparatus comprising:

a body cavity probe that is inserted into a body cavity in a subject;
insertion shape creation means for creating the insertion shape of the body cavity probe;

3-dimensional image creation means for creating a 3-dimensional image of a human body from 3-dimensional data on the human body; and

synthesis means for synthesizing the insertion shape and the 3-dimensional image,

the body cavity probe comprising a rigid portion having image signal acquisition means fixed on a side thereof which is inserted into the body cavity to acquire a signal from which an image of the interior of the subject is created and a flexible portion located closer to a proximal end than the rigid portion, and

comprising:

image creation means for creating a real-time image of the interior of the subject from the signal acquired by the image signal acquisition means;

image position and orientation detecting device the position of which is fixed to the rigid portion;

a plurality of insertion shape detecting devices provided along the flexible portion;

a subject detecting device that is able to come into contact with the subject;

detection means for detecting six degrees of freedom for the position and orientation of the image position and orientation detecting device, the position of each of the plurality of insertion shape detecting devices, and the position or orientation of the subject detecting device and outputting corresponding detection values; and

image index creation means for creating image indices indicating the position and orientation of the real-time image of the interior of the subject created by the image creation means,

the synthesis means synthesizing the insertion shape, the image indices, and the 3-dimensional image on the basis of the detection values outputted by the detection means to create a 3-dimensional guide image that guides the positions and orientations of the flexible portion and the real-time image with respect to the subject.

2. The body cavity probe apparatus according to claim 1, further comprising contact means containing the subject detecting device fixed thereto and simultaneously or sequentially coming into contact with predetermined positions of the subject,

the detection means outputting the predetermined positions based on the contact positions of the subject detecting device, as detection values,

the synthesis means synthesizing the positions of the insertion shape, the image indices, and the 3-dimensional image on the basis of the detection values outputted by the detection means to create a 3-dimensional guide image that guides the positions and orientations of the flexible portion and the real-time image with respect to the subject.

3. The body cavity probe apparatus according to claim 2, wherein the flexible portion has a tubular channel, and the contact means fixes and contains the subject detecting device at a distal end thereof and is inserted through the channel to come into contact with the predetermined positions in the body cavity in the subject.

4. The body cavity probe apparatus according to claim 1, wherein the 3-dimensional image creation means has extraction means for extracting an organ or a vessel from 3-dimensional data obtained from the subject through image pickup, and

the 3-dimensional image creation means creates a 3-dimensional image expressing the shape and location of the organ or vessel of the subject, from the organ or vessel extracted by the extraction means, and the synthesis means synthesizes the insertion shape, the image indices, and the 3-dimensional image on the basis of the detection values outputted by the detection means to create a 3-dimensional guide image that guides the positions and orientations of the flexible portion and the real-time image with respect to the subject.

5. The body cavity probe apparatus according to claim 1, wherein the image signal acquisition means is an image pickup device that picks up an image of the interior of the subject to output a video signal, and

the image creation means creates an optical image from the video signal as the real-time image.

6. The body cavity probe apparatus according to claim 1, wherein the image signal acquisition means is an ultrasonic transducer that transmits and receives an ultrasonic wave to and from the interior of the subject to output an echo signal, and

the image creation means creates an ultrasonic tomogram from the echo signal as the real-time image.

7. The body cavity probe apparatus according to claim 1, wherein the image position and orientation detecting device, the insertion shape detecting devices, and the subject detecting device are magnetic field generators or magnetic field detectors, and

the detection means uses a magnetic field to perform the detection.

**FIG.1**

1

DISPLAY DEVICE — 14

ANALOG VIDEO SIGNAL

OPTICAL IMAGE DATA

ULTRASONIC TOMOGRAM DATA

OPTICAL OBSERVATION DEVICE — 3

ULTRASONIC OBSERVATION DEVICE — 4

CCD SIGNAL

ECHO SIGNAL

SCAN CONTROL SIGNAL

IMAGE PROCESSING DEVICE — 11

REFERENCE IMAGE DATA

X-RAY 3-DIMENSIONAL HELICAL COMPUTER TOMOGRAPHY SYSTEM — 15

REFERENCE IMAGE DATA

3-DIMENSIONAL MAGNETIC RESONANCE IMAGING SYSTEM — 16

17

33

ULTRASONIC BEAM

$V_{12}$  $V_3$  V

23

30

22

2

32

46

21

25 26 31

29a 29 24 42 45

POSITIONAL ELECTRIC SIGNAL

8

44

7

36

35

34

POSITION AND ORIENTATION DATA

A/D  A/D  A/D

9c 9b 9a

9

ALTERNATING MAGNETIC FIELD

TRANSMISSION ANTENNA

6

POSITION AND ORIENTATION CALCULATION DEVICE — 5

12

13

65

13α 13β 13γ

66

# FIG.2

RIGHT ANTERIOR
SUPERIOR ILIAC SPINE

XIPHOID
PROCESS

SPINOUS PROCESS OF
VERTEBRAL BODY

LEFT ANTERIOR
SUPERIOR
ILIAC SPINE

37

7

7

7

7

PILLOW

BED

38

# FIG.3

42    41    8

SIGNAL
LINE

TO POSITION AND
ORIENTATION
CALCULATION
DEVICE 5

43

# FIG.4

OPTICAL OBSERVATION DEVICE *3*

ULTRASONIC OBSERVATION DEVICE *4*

OPTICAL IMAGE DATA

ULTRASONIC TOMOGRAM DATA

SCAN CONTROL SIGNAL

*61*

MIXING CIRCUIT — MIXTURE DATA

(VENTRAL SIDE) (CAUDAL SIDE)

3-DIMENSIONAL GUIDE IMAGE DATA

3-DIMENSIONAL GUIDE IMAGE CREATION CIRCUIT A

3-DIMENSIONAL GUIDE IMAGE CREATION CIRCUIT B

*60*

DISPLAY DEVICE *14*

β

γ

α

DISPLAY CIRCUIT *62a* *62*

ANALOG VIDEO SIGNAL

*12*

KEYBOARD *13*

*63*

CONTROL CIRCUIT

ROTATION INSTRUCTION SIGNAL

ROTATION CONVERSION CIRCUIT *59*

SYNTHETIC 3-DIMENSONAL DATA

POSITION AND ORIENTATION CALCULATION DEVICE *5*

POSITION AND ORIENTATION DATA

*51*

MATCHING CIRCUIT

POSITION AND ORIENTATION MAPPING DATA

*52*

IMAGE INDEX CREATION CIRCUIT

IMAGE INDEX DATA

*53*

INSERTION SHAPE CREATION CIRCUIT

INSERTION SHAPE DATA

SYNTHESIS CIRCUIT VM *58a* *58*

3-DIMENSONAL BODY IMAGE DATA

3-DIMENSIONAL BODY IMAGE CREATION CIRCUIT *57*

VOXEL DATA

BODY SURFACE FEATURE POINT COORDINATES

BODY CAVITY FEATURE POINT COORDINATES

NETWORK *17*

REFERENCE IMAGE DATA

*54*

COMMUNICATION CIRCUIT

REFERENCE IMAGE DATA

*55*

INTERPOLATION CIRCUIT VM *56a* *56*

*11*

EP 1 872 707 A1

# FIG.5

# FIG.6

VOXEL SPACE

y'

z'

O'

x'

VOXEL

# FIG.7

ULTRASONIC
TOMOGRAM IMAGE

$V_{12}$

V

$V_3$

z

31

O"

θ

k

φ

j

O

y

i

ψ

x

H

6

TRANSMISSION ANTENNA

# FIG.8

# FIG.9

# FIG.10

MARKER
CREATION

# FIG.11

POSITION OF INSERTION
SHAPE DETECTION COIL

POSITION OF POSITION
AND ORIENTATION
DETECTION COIL

$(x4, y4, z4)$

$(x3, y3, z3)$

$(x2, y2, z2)$

$(x1, y1, z1)$

$(x0, y0, z0)$

z

y

O

x

INTERPOLATION AND
MARKER CREATION

Ms

Mc

z'

y'

O'

x'

# FIG.12

CAUDAL SIDE

AORTA (RED)

SUPERIOR MESENTERIC VEIN (RED)

PANCREAS (GREEN)

CRANIAL SIDE

# FIG.13

$Mc$

$z'$

$Mu$

$Md$

$Mt$

$Ms$

$y'$

$O'$

$x'$

# FIG.14

# FIG.15

*Mc*

*Mu*

*Md*

*Mt*

*Ms*

LEFT

RIGHT

PANCREAS
(GREEN)

AORTA
(RED)

SUPERIOR
MESENTERIC VEIN
(RED)

# FIG.16

**DISPLAY SCREEN**
**(3-DIMENSIONAL GUIDE IMAGE, ULTRASONIC TOMOGRAM IMAGE)**

3-DIMENSIONAL GUIDE IMAGE (VENTRALLY OBSERVED)

*Mu*

**ULTRASONIC TOMOGRAM IMAGE**

MOVED IN CONJUNCTION WITH MOVEMENT OF RADIAL SCAN SURFACE

3-DIMENSIONAL GUIDE IMAGE (CAUDALLY OBSERVED)

PANCREAS (GREEN)   AORTA (RED)   DUODENUM WALL

*Mu*

EP 1 872 707 A1

# FIG.17

START

S1 — SPECIFY BODY SURFACE
FEATURE POINTS AND BODY
CAVITY FEATURE POINTS
ON REFERENCE IMAGE DATA

S2 — FIX BODY SURFACE
DETECTION COIL TO SUBJECT

S3 — CALCULATE
CORRECTION VALUE

S4 — CREATE/DISPLAY ULTRASONIC
TOMOGRAM IMAGE AND
3-DIMENSIONAL GUIDE IMAGE

END

# FIG.18

SPECIFICATION OF BODY SURFACE FEATURE POINTS AND BODY CAVITY FEATURE POINTS ON REFERENCE IMAGE DATA

S1-1   DEPRESS DISPLAY SWITCHING KEY α  ←— DISPLAY SWITCHING KEY 13α

S1-2   SPECIFY REFERENCE IMAGE DATA  ←— MOUSE 12 / KEYBOARD 13

S1-3   DISPLAY REFERENCE IMAGE DATA

S1-4   SPECIFY BODY SURFACE FEATURE POINTS ON REFERENCE IMAGE DATA  ←— MOUSE 12 / KEYBOARD 13

S1-5   SPECIFY BODY CAVITY FEATURE POINTS P" ON REFERENCE IMAGE DATA  ←— MOUSE 12 / KEYBOARD 13

S1-6   STORE COORDIANTES OF EACH FEATURE POINT IN VOXEL SPACE

RETURN

42

# FIG.19

CALCULATION OF
CORRECTION VALUE

S3-1 — DEPRESS DISPLAY
SWITCHING KEY β ← DISPLAY SWITCHING KEY 13β

S3-2 — DISPLAY OPTICAL IMAGE

S3-3 — INSERT RIGID AND
FLEXIBLE PORTIONS
INTO BODY CAVITY

S3-4 — MOVE RIGID PORTION
TO SEARCH FOR BODY
CAVITY FEATURE POINT

S3-5 — PROJECT BODY CAVITY
CONTACT PROBE FROM
PROJECTION PORT TO
CONTACT PROBE WITH
BODY CAVITY FEATURE
POINT

S3-6 — DEPRESS BODY CAVITY
FEATURE POINT
SPECIFICATION KEY ← FEATURE POINT SPECIFICATION KEY 65

S3-7 — LOAD POSITION AND
ORIENTATION DATA ← POSITION AND ORIENTATION
CALCULATION DEVICE 5

S3-8 — CREATE FIRST
CONVERSION EQUATION
EXPRESSING FIRST MAP
FROM COORDINATES OF
BODY SURFACE
FEATURE POINTS

CALCULATE VECTOR P'P" — S3-10

S3-9 — MAP BODY CAVITY
FEATURE POINT P TO
POINT P' IN VOXEL
SPACE BY FIRST
CONVERSION EQUATION

STORE VECTOR P'P"
AS CORRECTION VALUE — S3-11

RETURN

# FIG.20

DISPLAY SCREEN
(OPTICAL IMAGE)

DUODENUM PAPPILA P

BODY CAVITY
CONTACT PROBE 8

# FIG.22

Mu
Md
Mt

Mc

Ms

PANCREAS (GREEN)

AORTA (RED)

SUPERIOR MESENTERIC VEIN (RED)

# FIG.21

```
        ( CREATION AND
          DISPLAY OF ULTRASONIC
          TOMOGRAM IMAGE AND
          3-DIMENSIONAL
          GUIDE IMAGE )
                │
                ▼
S4-1 ──┌─────────────────────┐
       │  DEPRESS DISPLAY    │◄── DISPLAY SWITCHING KEY 13γ
       │  SWITCHING KEY γ    │
       └─────────────────────┘
                │
                ▼
S4-2 ──┌─────────────────────┐
       │  DEPRESS SCAN       │◄── SCAN CONTROL KEY 66
       │  CONTROL KEY        │
       └─────────────────────┘
                │
                ▼
S4-3 ──┌─────────────────────┐
       │  START RADIAL SCAN  │
       └─────────────────────┘
                │
                ▼
S4-4 ──/ LOAD ULTRASONIC    /◄── ULTRASONIC OBSERVATION DEVICE 4
       / TOMOGRAM DATA      /
                │
                ▼
S4-5 ──/ LOAD POSITION AND  /◄── POSITION AND ORIENTATION
       / ORIENTATION DATA   /     CALCULATION DEVICE 5
                │
                ▼
S4-6 ──┌─────────────────────┐
       │ COMBINE FIRST       │
       │ CONVERSION          │
       │ EQUATION WITH       │          ┌────────────┐
       │ PARALLEL            │          │            │
       │ MOVEMENT BASED ON   │          ▼        S4-10
       │ VECTOR P'P" TO NEWLY│      ◇ RADIAL SCAN ◇── NO ──┐
       │ CREATE SECOND       │        FINISHED?            │
       │ CONVERSION EQUATION │          │                  │
       │ EXPRESSING SECOND   │         YES                 │
       │ MAP                 │          ▼                  │
       └─────────────────────┘      ( RETURN )             │
                │                                           │
                ▼                                           │
S4-7 ──┌─────────────────────┐                             │
       │ CONVERT POSITION AND│                             │
       │ ORIENTATION DATA    │                             │
       │ INTO POSITION AND   │                             │
       │ ORIENTATION         │                             │
       │ MAPPING DATA        │                             │
       └─────────────────────┘                             │
                │                                           │
                ▼                                           │
S4-8 ──┌─────────────────────┐                             │
       │ CREATE 3-DIMENSIONAL│                             │
       │ GUIDE IMAGE DATA    │                             │
       └─────────────────────┘                             │
                │                                           │
                ▼                                           │
S4-9 ──( DISPLAY ULTRASONIC                                │
          TOMOGRAM IMAGE AND                                │
          3-DIMENSIONAL                                     │
          GUIDE IMAGE )───────────────────────────────────┘
```

# FIG.23

AORTA
(REAR, LIGHT RED)

Mu
Md
Mt

Mc

Ms

SUPERIOR
MESENTERIC VEIN
(REAR, LIGHT RED)

PANCREAS
(FRONT, DARK GREEN)

PANCREAS
(REAR, LIGHT GREEN)

AORTA
(FRONT, DARK RED)

SUPERIOR MESENTERIC VEIN
(FRONT, DARK RED)

# FIG.24

AORTA
(REAR, LIGHT RED)

Mu
Md
Mt

Mc

Ms

SUPERIOR
MESENTERIC VEIN
(REAR, LIGHT RED)

PANCREAS
(ON ULTRASONIC TOMOGRAM
IMAGE MARKER, DARK GREEN)

PANCREAS
(REAR, LIGHT GREEN)

AORTA
(ON ULTRASONIC TOMOGRAM
IMAGE MARKER, DARK RED)

SUPERIOR MESENTERIC VEIN
(ON ULTRASONIC TOMOGRAM
IMAGE MARKER, DARK RED)

# FIG.25

AORTA
(REAR, LIGHT RED)

*Mu*

*Md*

*Mt*

*Mc*

SUPERIOR
MESENTERIC VEIN
(REAR, LIGHT RED)

*Ms*

LEFT

RIGHT

PANCREAS
(REAR, LIGHT GREEN)

PANCREAS
(FRONT, DARK GREEN)

AORTA
(FRONT, DARK RED)

SUPERIOR MESENTERIC VEIN
(FRONT, DARK RED)

# FIG.26

OPTICAL OBSERVATION DEVICE *3*

OPTICAL IMAGE DATA

*61*

MIXING CIRCUIT — MIXTURE DATA

(VENTRAL SIDE) (CAUDAL SIDE)

3-DIMENSIONAL GUIDE IMAGE DATA

3-DIMENSIONAL GUIDE IMAGE CREATION CIRCUIT A

3-DIMENSIONAL GUIDE IMAGE CREATION CIRCUIT B

*60*

DISPLAY CIRCUIT *62a*

*62*

β

γ

α

*14*

DISPLAY DEVICE

ANALOG VIDEO SIGNAL

*12*

KEYBOARD *13*

*63*

CONTROL CIRCUIT

ROTATION INSTRUCTION SIGNAL

ROTATION CONVERSION CIRCUIT *59*

POSITION AND ORIENTATION DATA

POSITION AND ORIENTAATION CALCULATION DEVICE *5*

*51*

MATCHING CIRCUIT

POSITION AND ORIENTATION MAPPING DATA

*52*

IMAGE INDEX CREATION CIRCUIT

IMAGE INDEX DATA

SYNTHETIC 3-DIMENSONAL DATA

SYNTHESIS CIRCUIT VM *58a* *58*

INSERTION SHAPE CREATION CIRCUIT

*53*

INSERTION SHAPE DATA

3-DIMENSONAL BODY IMAGE DATA

3-DIMENSIONAL BODY IMAGE CREATION CIRCUIT *57*

VOXEL DATA

*11*

BODY SURFACE FEATURE POINT COORDINATES

BODY CAVITY FEATURE POINT COORDINATES

*54*

NETWORK *17*

REFERENCE IMAGE DATA

COMMUNICATION CIRCUIT

REFERENCE IMAGE DATA

*55*

INTERPOLATION CIRCUIT VM *56a* *56*

EP 1 872 707 A1

# FIG.27

Mc

OPTICAL IMAGE VISUAL
FIELD DIRECTION MARKER

Ms

CAUDAL
SIDE

CRANIAL
SIDE

BRONCHUS

CARINA a

OPTICAL IMAGE UP
DIRECTION MARKER

CARINA b

# FIG.28

Mc

OPTICAL IMAGE VISUAL
FIELD DIRECTION MARKER

Ms

LEFT

RIGHT

BRONCHUS

CARINA a

CARINA b

OPTICAL IMAGE UP
DIRECTION MARKER

# FIG.29

DISPLAY SCREEN
(3-DIMENSIONAL GUIDE IMAGE, ULTRASONIC TOMOGRAM IMAGE)

3-DIMENSIONAL GUIDE IMAGE (VENTRALLY OBSERVED)

OPTICAL IMAGE

MOVED AND DEFORMED
IN CONJUNCTION WITH
MOVEMENT OF FLEXIBLE
AND RIGID PORTIONS

3-DIMENSIONAL GUIDE IMAGE (CAUDALLY OBSERVED)

CARINA b    CARINA a

EP 1 872 707 A1

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2006/057296 A (OLYMPUS CORP [JP]; KAWASHIMA TOMONAO [JP]; KOMURO MASAHIKO) 1 June 2006 (2006-06-01) * abstract * * figures 1-5,8-16,18-21,23 * | 1-7 | INV. A61B1/005 A61B5/06 A61B8/12 |
| E | -& EP 1 815 797 A (OLYMPUS CORP [JP]) 8 August 2007 (2007-08-08) * abstract * * paragraph [0047] - paragraph [0324] * * figures 1-5,8-16,18-21,23 * | 1-7 | ADD. A61B5/055 A61B6/03 |
| Y | EP 1 354 557 A (OLYMPUS OPTICAL CO [JP]) 22 October 2003 (2003-10-22) * abstract * * paragraph [0018] - paragraph [0132] * * figures 1,2,4-9 * | 1-7 | |
| D,A | EP 1 543 776 A (OLYMPUS CORP [JP]) 22 June 2005 (2005-06-22) * the whole document * | 1-7 | |
| D,A | JP 2002 306403 A (OLYMPUS OPTICAL CO) 22 October 2002 (2002-10-22) * abstract * * figures 1-3,6,7 * | 1-7 | TECHNICAL FIELDS SEARCHED (IPC) A61B |

The present search report has been drawn up for all claims

| Place of search | Date of completion of the search | Examiner |
|---|---|---|
| Munich | 12 October 2007 | ARTIKIS, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 01 1871

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2007

| Patent document cited in search report | | Publication date | Patent family member(s) | | Publication date |
|---|---|---|---|---|---|
| WO 2006057296 | A | 01-06-2006 | EP | 1815797 A1 | 08-08-2007 |
| | | | JP | 2006149481 A | 15-06-2006 |
| EP 1815797 | A | 08-08-2007 | JP | 2006149481 A | 15-06-2006 |
| | | | WO | 2006057296 A1 | 01-06-2006 |
| EP 1354557 | A | 22-10-2003 | JP | 2003305044 A | 28-10-2003 |
| | | | US | 2003199756 A1 | 23-10-2003 |
| EP 1543776 | A | 22-06-2005 | WO | 2004028374 A1 | 08-04-2004 |
| | | | JP | 2004113629 A | 15-04-2004 |
| | | | US | 2005203417 A1 | 15-09-2005 |
| JP 2002306403 | A | 22-10-2002 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004113629 A

- JP 2002306403 A